# EUROPEAN PATENT APPLICATION

(11) **EP 2 452 936 A1**
(43) Date of publication of application: **16.05.2012**
(21) Application number: 11193612.6
(22) Date of filing: 14.07.2006
(51) Int. Cl.: C07D 249/04, C07D 249/06, C07D 403/06

(54) **Compositions and methods for coupling a plurality of compounds to a scaffold**

(30) Priority: 14.07.2005 US 699985 P; 13.07.2006 US 486646
(62) Divisional of application: 06787247.3
(71) Applicant: The Scripps Research Institute, La Jolla, CA 92037 (US)
(72) Inventor: Finn, M. G., San Diego, CA 92129-4134 (US); Gupta, Sayam, Sen, Irvine, CA 92617 (US); Sharpless, Karl, Berry, La Jolla, CA 92037 (US); Fokin, Valery, V., Oceanside, CA 92054-6130 (US)
(74) Representative: Chapman, Desmond Mark

(57) **Abstract**

Compositions and methods are provided for coupling a plurality of compounds to a scaffold. Compositions and methods are further provided for catalyzing a reaction between at least one terminal alkyne moiety and at least one azide moiety, wherein one moiety is attached to the compound and the other moiety is attached to the scaffold, forming at least one triazole thereby.

## Description

### STATEMENT OF GOVERNMENT SUPPORT

This invention was made with Government support of grant numbers EB00432 and N01-CO-27181 from the National Institutes of Health. The Government has certain rights in this invention.

### CROSS-REFERENCE TO RELATED APPLICATIONS

This application claims the benefit of U.S. Provisional Application No. 60/699,985, filed July 14, 2005, and U.S. Application entitled "COMPOSITIONS AND METHODS FOR COUPLING A PLURALITY OF COMPOUNDS TO A SCAFFOLD," filed July 13, 2006, by Express Mail No. EV 670672044 US, the entire disclosures of which are incorporated herein by reference.

### FIELD

The present invention relates to compositions and methods for coupling a plurality of compounds to a scaffold. The invention further provides compositions and methods for catalyzing a reaction between at least one terminal alkyne moiety and at least one azide moiety, wherein one moiety is attached to the compound and the other moiety is attached to the scaffold, forming at least one triazole thereby.

### BACKGROUND

The polyvalent clustering of carbohydrate derivatives based on linear polymers and dendrimers has proven to be an effective tool in the study of carbohydrate-based cellular processes and is a useful strategy in the development of therapeutic agents. Spaltenstein and Whitesides, J. Am. Chem. Soc. 113: 686, 1991; Gordon et al., Nature 392: 30, 1998; Griffith et al., J. Am. Chem. Soc. 126: 1608, 2003; Owen et al., Org. Lett. 4: 2293, 2002; Gestwicki et al., Chem. Biol. 9: 163, 2002; Gestwicki and Kiessling, Nature 415: 81, 2002; Cairo et al., J. Am. Chem. Soc. 124: 1615, 2002; Nagasaki et al., Biomacromolecules 2: 1067, 2001; Woller et al., J. Am. Chem. Soc. 125: 8820, 2003; Woller and Cloninger, Org. Lett. 4: 7, 2002; Thoma et al., Angew. Chem. Int. Ed. 41: 3195, 2002; Roy et al., J. Am. Chem. Soc. 123: 1809, 2001; Ortega-Caballero et al., J. Org. Chem. 66: 7786, 2001; Zanini and Roy, J. Org. Chem. 63: 3486, 1998; Pagé and Roy, Bioconj. Chem. 8: 714, 1997; Pagé et al., Chem. Commun., 1913, 1996; Roy et al., J. Chem. Soc., Chem. Commun., 1869, 1993; Bader et al., Angew. Chem. Int. Ed. Engl. 20: 91, 1981; Matrosovich, FEBS Lett. 252: 1, 1989; Kamitakahara et al., Angew. Chem. Int. Ed. 37: 1524, 1998. Dense clusters of carbohydrates can be formed by arraying an end-functionalized glycopolymer to a biocompatible scaffold such as a protein. Such polymers have been recently prepared by cyanoxyl-mediated free radical polymerization (employing initiators bearing amine, carboxylic acid, hydrazide, or biotin moieties, with subsequent protein attachment by biotin-avidin binding) and atom transfer radical polymerization (ATRP; side-chain PEG or poly(HEMA) polymers containing N-hydroxysuccinamide or pyridyl disulphide end groups, with protein attachment to lysozyme and BSA). Hou et al., Bioconj. Chem. 15: 954, 2004; Sun et al., J. Am. Chem. Soc. 124: 7258, 2002; Bontempo et al., J. Am. Chem. Soc. 126: 15372, 2004; Lecolley et al., Chem. Commun., 2026, 2004.

Methods for bioconjugation by attaching molecules to biological structures has been reviewed in "Bioconjugate Techniques" by Greg T. Hermanson, Academic Press, 1996, ISBN 0-12-342336-8. A further method for bioconjugation utilizes "native chemical ligation." For native chemical ligation (NCL), two fully unprotected synthetic peptide fragments are chemically ligated under neutral aqueous conditions with the formation of a normal (native) peptide bond at the ligation site. The NCL reaction requires an N-terminal cysteine on a peptide or protein chain and is therefore limited in its application. Gentle et al., Bioconjugate Chem. 15: 658-663, 2004; Muir, Synlett 6: 733-740, 2001.

Bioconjugation requires the most active and selective organic reactions that are compatible with water as a solvent. Improvements in the above methods are needed to allow the maximum possible range of reaction partners and greater reaction rates selectivities. Organic azides have achieved wide application due to their inert nature toward biological molecules and their participation in the Staudinger ligation with phosphine-esters and the 1,3-dipolar cycloaddition reactions with alkynes. Lemieux and Bertozzi, TIBTECH 16: 506, 1998; Saxon et al., Org. Lett. 2: 2141, 2000; Saxon and Bertozzi, Science 287: 2007, 2000; Kiick et al., Proc. Nat. Acad. Sci. USA 99: 19, 2002; Wang et al., J. Am. Chem. Soc. 125: 3192, 2003; Speers et al., J. Am. Chem. Soc. 125: 4686, 2003; Link and Tirrell, J. Am. Chem. Soc. 125: 11164, 2003; Link et al., J. Am. Chem. Soc. 126: 10598, 2004. The latter process can be extraordinarily fast and versatile in demanding bioconjugation applications under dilute conditions. There is a version of the azide-alkyne reaction that does not require metal catalyst and is much slower, but it also has been used for bioconjugation. This is done by making the alkyne more reactive, and is therefore limited to such molecules. Prescher and Bertozzi, J. Am. Chem. Soc. 126: 15046, 2004. It has also been used in a wide variety of other applications, including the creation of small dendrimer-style polyvalent carbohydrate assemblies. Wang et al., J. Am. Chem. Soc. 125: 3192, 2003; Lewis et al., J. Am. Chem. Soc. 126: 9152, 2004; Gupta *et al.,* unpublished results; Calvo-Flores et al., Org. Lett., 2: 2499, 2000; Pérez-Balderas et al., Org. Lett., 5: 1951, 2003; Bodine et al., J. Am. Chem. Soc. 126: 1638, 2004. Atom-transfer radical polymerization (ATRP) can be used to create polymer chains bearing multiple carboydrate groups. Since Cu(I) complexes catalyze both the ATRP and azide-alkyne cycloaddition (AAC) reactions, their combination is logical. Matyjaszewski et al., Macromolecules 31: 5967, 1998; Xia et al., Macromolecules 31: 5958, 1998; Matyjaszewski et al., Macromolecules 34: 430, 2001; Rostovtsev et al., Angew. Chem. Int. Ed., 41: 2596, 2002; Tornøe et al., J. Org. Chem., 67: 3057, 2002.

Viruses are intriguing scaffolds for the polyvalent presentation of functional structures. Chemistry-based studies have included the organization of inorganic materials in or around virus cages, the organization of viruses on surfaces, and the chemical conjugation of organic compounds to virus coat proteins. Klem et al., J. Am. Chem. Soc. 125: 10806, 2003; Douglas et al., Adv. Mater. 14: 415, 2002; Douglas and Young, Nature 393: 152, 1998; Shenton et al., Adv. Mater. 11: 253, 1999; Douglas and Young, Adv. Mater. 11: 679, 1999; Whaley et al., Nature 405: 665, 2000; Lee et al., Science 296: 892, 2002; Mao et al., Science 303: 213, 2004; Wang et al., Angew. Chem. Int. Ed. 41: 459, 2002; Wang et al., Chem. Biol. 9: 805, 2002; Wang et al., Chem. Biol. 9: 813, 2002; Wang et al., Bioconj. Chem. 14: 38, 2003; Meunier et al., Chem. Biol. 11: 319, 2004; Gillitzer et al., Chem. Commun., 2390, 2002; Flenniken et al., Nano Lett. 3: 1573, 2003; Hooker et al., J. Am. Chem. Soc. 2004: 3718, 2004; Wu et al., Bioconj. Chem. 6: 587, 1995. Work in this area has comprised a broad exploration of virus particles as chemical building blocks, focused on cowpea mosaic virus (CPMV) as a prototype. This plant virus can be made and purified in large quantities, is structurally characterized to near-atomic resolution, is stable to a variety of conditions compatible with both hydrophobic and hydrophilic molecules, and can be manipulated at the genetic level to introduce mutations at desired positions. One goal is to bring new functions to virus particles by attaching functional molecules to the capsid protein, thereby generating novel species with diagnostic and therapeutic applications. Attachment of single carbohydrate compounds to CPMV residues produces a dendrimer-like display with polyvalent lectin-binding properties. Raja et al., ChemBioChem 4: 1348, 2003. CPMV has been derivatized with poly(ethylene glycol) (PEG) to give well-controlled loadings of polymer on the outer surface of the coat protein assembly. Raja et al., Biomacromolecules 4: 472, 2003. The resulting conjugates displayed altered physical properties and reduced immunogenicities, consistent with previous reports of PEGylated adenovirus vectors. Fisher et al., Polym. Prepr. (Am. Chem. Soc., Div. Polym. Chem.) 41, 1012, 2000; O'Riordan et al., Hum. Gene Ther. 10: 1349, 1999; Marlow et al., Proc. Int. Symp. Controlled Release Bioact. Mater. 26: 555, 1999. The need to make covalent attachments to virus particles is an illustrative application of bioconjugation. Covalent bond formation to proteins is made difficult by multiple unprotected functional groups on proteins and normally low concentrations. A need exists in the art for a more effective conjugation process to increase the efficiency of conjugation and increase the number of functional molecules that can be attached to each viral particle.

### SUMMARY

Compositions and methods are provided for coupling a plurality of compounds to a scaffold. The scaffold can be a biological or non-biological surface. The scaffold includes, for example, a solid surface, a protein, a glass bead, or a polymer bead. The scaffold further includes a protein or nucleoprotein nanoparticle, including viruses and other large assemblies. The scaffold further includes, for example, a protein on a viral nanoparticle. The compound coupled to the scaffold includes, for example, a small molecule, a metal complex, a polymer, a carbohydrate, a protein, or a polynucleotide. Compositions and methods for Cu(I)-catalyzed atom transfer radical polymerization (ATRP) and azide-alkyne cycloaddition reactions together provide a versatile method for the synthesis of end-functionalized compounds, e.g., glycopolymers, proteins, polynucleotides, or metal complexes, and their attachment to a scaffold, e.g., a suitably modified viral protein scaffold. Further compositions and methods are provided for the construction of azide-terminated glycopolymers by ATRP, their end-labeling with fluorophores, and the subsequent conjugation of these compounds to virus particles in high yield for purposes of polyvalent binding to cell-surface lectins. The compositions and methods for covalently coupling a plurality of compounds to a scaffold provide a coupling reaction to a range of biological and non-biological surfaces having increased efficiency and selectivity.

A method for coupling a compound to a scaffold is provided comprising catalyzing a reaction between at least one terminal alkyne moiety on the compound, and at least one azide moiety on the scaffold forming at least one triazole thereby, the catalysis being effected by addition of a metal ion in the presence of a ligand for the metal ion, and the scaffold having a plurality of such azide moieties, such that a plurality of compound molecules can be coupled with the scaffold. In one aspect, the ligand is monodentate, bidentate, or multidentate. In a further aspect, the metal is Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg.

In a further aspect, the metal is heterogeneous copper, metallic copper, copper oxide, or copper salts. The method further provides catalyzing the reaction by addition of Cu(I). The method further provides catalyzing the reaction by addition of Cu(II) in the presence of a reducing agent for reducing the Cu(II) to Cu(I), *in situ.* The method further provides catalyzing the reaction by addition of Cu(0) in the presence of an oxidizing agent for oxidizing the Cu(0) to Cu(I), *in situ.*

The scaffold can be a biological or non-biological surface. In one aspect, the scaffold is a solid surface, a protein, a protein aggregate, or a nucleoprotein. The scaffold further includes a protein nanoparticle or nucleoprotein nanoparticle, including viruses, viral nanoparticles, vault protein, dendrimer, or other large assemblies. In a detailed aspect, the virus or viral nanoparticle is a cowpea mosaic virus nanoparticle. The scaffold can be a protein aggregate, for example, keyhole limpet hemocyanin or tetanus toxin.

The scaffold can be a non-biological surface, for example, a particle, glass bead, metal nanoparticle, gold particle, polymer bead, membrane, electrode, or porous materials such as fiber-based materials, zeolites, clays, or controlled-pore glass. The particle can be a paramagnetic particle, semiconductor nanoparticle, or quantum dot.

In a further aspect, the compound is a small molecule, a metal complex, a polymer, a carbohydrate, a protein, or a polynucleotide. In a detailed aspect, the compound is transferrin, an RGD-containing polypeptide, a protective antigen of anthrax toxin, polyethylene glycol, or folic acid.

The method further provides coupling a multiplicity of compound molecules per scaffold. The method further provides coupling a multiplicity of compound molecules per viral nanoparticle. In a further detailed aspect, the method provides coupling 100 or more compound molecules per viral nanoparticle. In a further detailed aspect, the method provides coupling 150 or more compound molecules per viral nanoparticle. In a further detailed aspect, the method provides coupling 200 or more compound molecules per viral nanoparticle.

A method for coupling a compound to a scaffold is provided comprising catalyzing a reaction between at least one azide moiety on the compound, and at least one terminal alkyne moiety on the scaffold forming at least one triazole thereby, the catalysis being effected by addition of a metal ion in the presence of a ligand for the metal ion, and the scaffold having a plurality of such terminal alkyne moieties, such that a plurality of compound molecules can be coupled with the scaffold. In one aspect, the ligand is monodentate, bidentate, or multidentate. In a further aspect, the metal is Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg.

In a further aspect, the metal is heterogeneous copper, metallic copper, copper oxide, or copper salts. The method further provides catalyzing the reaction by addition of Cu(I). The method further provides catalyzing the reaction by addition of Cu(II) in the presence of a reducing agent for reducing the Cu(II) to Cu(I), *in situ.* The method further provides catalyzing the reaction by addition of Cu(0) in the presence of an oxidizing agent for oxidizing the Cu(0) to Cu(I), *in situ.*

In one aspect, the scaffold is a solid surface, a protein, glass bead, or polymer bead. In a further aspect, the scaffold is a viral nanoparticle In a detailed aspect, the viral nanoparticle is a cowpea mosaic virus nanoparticle. In a further aspect, the compound is a small molecule, a metal complex, a polymer, a carbohydrate, a protein, or a polynucleotide. In a detailed aspect, the compound is transferrin, an RGD-containing polypeptide, a protective antigen of anthrax toxin, polyethylene glycol, or folic acid.

The method further provides coupling a multiplicity of compound molecules per scaffold. The method further provides coupling a multiplicity of compound molecules per viral nanoparticle. In a further detailed aspect, the method provides coupling 100 or more compound molecules per viral nanoparticle. In a further detailed aspect, the method provides coupling 150 or more compound molecules per viral nanoparticle. In a further detailed aspect, the method provides coupling 200 or more compound molecules per viral nanoparticle.

A method is provided comprising catalyzing a reaction between at least one terminal alkyne moiety on a first reactant and at least one azide moiety on a second reactant forming at least one triazole thereby, the catalysis being effected by addition of a metal in the presence of a ligand for the metal ion, and the first reactant having a plurality of terminal alkyne moieties such that a plurality of second reactants can be coupled to the first reactant, or the second reactant having a plurality of azide moieties such that a plurality of first reactants can be coupled to the second reactant. In one aspect, the ligand is monodentate, bidentate, or multidentate. In a further aspect, the metal is Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg.

In a further aspect, the metal is heterogeneous copper, metallic copper, copper oxide, or copper salts. The method further provides catalyzing the reaction by addition of Cu(I). The method further provides catalyzing the reaction by addition of Cu(II) in the presence of a reducing agent for reducing the Cu(II) to Cu(I), *in situ*. The method further provides catalyzing the reaction by addition of Cu(0) in the presence of an oxidizing agent for oxidizing the Cu(0) to Cu(I), *in situ.*

In one aspect, the first reactant is a scaffold having a plurality of terminal alkyne moieties for coupling to the second reactant, and the second reactant is a compound with one or more azide moieties.

In another aspect, the second reactant is a scaffold having a plurality of azide moieties for coupling to the first reactant, and the first reactant is a compound with one or more terminal alkyne moieties.

### BRIEF DESCRIPTION OF THE DRAWINGS

Figure 1 shows synthesis of glycopolymers and virus-polymer conjugates.

Figure 2 shows (A) Size-exclusion FPLC (Superose 6) of wild-type CPMV and glycopolymer conjugate **9.** (B) FPLC on concanavalin-A Sepharose column of wild-type CPMV and virus-polymer conjugate **9.** (C) SDS-PAGE of **9** (lane 1) and WT-CPMV (lane 2). (D) Negative-stained TEM of **9** and enlarged TEM image of a WT-CPMV particle surrounded by **9.**

Figure 3 shows the construction of polymer-covered surfaces is made convenient by Cu^{I} catalysis of polymerization, end-labeling, and attachment steps.

Figure 4 shows a time course of agglutination for a mixture of con-A and **9.**

Figure 5 shows substrates used in CuAAC attachment to CPMV.

Figure 6 shows viral capsids labeled with alkynes or azides at surface-exposed lysine residues using standard N-hydroxysuccinimide (NHS) ester chemistry.

Figure 7 shows dependence of dye loading on reagent concentration.

Figure 8 shows SDS-PAGE of CPMV-(**13**)₉₀ and CPMV-(**5**)₁₁₀.

Figure 9 shows (A) size-exclusion FPLC of wild-type CPMV and CPMV-(**14**)ₙ. (B) SimplyBlue™-stained gel of wild-type CPMV, Tfn, and CPMV-(**14**)ₙ. (C) Negative-stained TEM of wild-type CPMV. (D) Negative-stained TEM of CPMV-(**14**)ₙ.

Figure 10 shows size-exclusion FPLC traces of CPMV-**5**.

Figure 11 shows a time course of agglutination monitored at 490 nm for a mixture of galectin-4 and CPMV-**8b** in phosphate-buffered saline.

Figure 12 shows size-exclusion FPLC of wild-type CPMV and CPMV-**13**.

Figure 13 shows Western blots of CPMV-**14** using polyclonal antibodies against CPMV or human Tfn.

Figure 14 shows examples of ligands, e.g., bidentate ligands.

### DETAILED DESCRIPTION

Compositions and methods are provided for coupling a plurality of compounds to a scaffold. The scaffold can be a biological or non-biological surface. The scaffold can be, for example, a solid surface, a protein, a glass bead, or a polymer bead. The scaffold further includes, for example, a protein on a viral nanoparticle. The compound coupled to the scaffold can be, for example, a small molecule, a metal complex, a polymer, a carbohydrate, a protein, or a polynucleotide. Compositions and methods are further provided for metal-catalyzed atom transfer radical polymerization (ATRP) and azide-alkyne cycloaddition reactions together to provide a versatile method for the synthesis of end-functionalized compounds, e.g., glycopolymers, proteins, polynucleotides, or metal complexes, and their attachment to a scaffold, *e.g.,* a suitably modified viral protein scaffold. The metal can be copper, *e.g.,* Cu(0), Cu(I), or Cu(II), in the presence of a ligand for the metal ion. The compositions and methods for covalently coupling a plurality of compounds to a scaffold provide a coupling reaction to a range of biological and non-biological surfaces having increased efficiency and selectivity.

Covalent bond formation to proteins is made difficult by their multiple unprotected functional groups and normally low concentrations. The water soluble sulfonated bathophenanthroline ligand 2 can be used to promote a highly efficient Cu(I)-mediated azide-alkyne cycloaddition (CuAAC) reaction for the chemoselective attachment of biologically relevant molecules to cowpea mosaic virus (CPMV) nanoparticles. The ligated substrates included complex sugars, peptides, poly(ethylene oxide) polymers, and the iron carrier protein transferring (Tfn), with successful ligation even for cases that were previously resistant to azide-alkyne coupling using the conventional ligand tris(triazolyl)amine (**1**). The use of 4―6 equiv of substrate was sufficient to achieve loadings of 60-115 molecules/virion in yields of 60-85%. Although it is sensitive to oxygen, the reliably efficient performance of the Cu-ligand•**2** system makes it a useful tool for demanding bioconjugation applications.

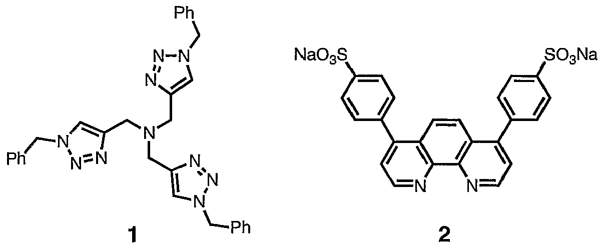

Compositions and methods are provided for catalyzing a reaction between at least one terminal alkyne moieties, and at least one azide moieties, wherein one moiety is attached to the compound and the other moiety is attached to the scaffold, forming at least one triazole thereby. A method for coupling a compound to a scaffold is provided comprising catalyzing a reaction between at least one terminal alkyne moieties attached to the compound, and at least one azide moieties attached to the scaffold, forming at least one triazole thereby, effecting catalysis by addition of a metal ion in the presence of a ligand, and providing a plurality of sites on the scaffold having azide moieties, such that a plurality of compound molecules can be coupled with the scaffold.. A further embodiment provides a method for coupling a compound to a scaffold is provided comprising catalyzing a ligation reaction between at least one terminal alkyne moieties attached to the scaffold, and at least one azide moieties attached to the compound, forming at least one triazole thereby, effecting catalysis by addition of a metal ion in the presence of a ligand, and providing a plurality of sites on the scaffold having terminal alkyne moieties, such that a plurality of compound molecules can be coupled with the scaffold..

It is to be understood that this invention is not limited to particular methods, reagents, compounds, compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a cell" includes a combination of two or more cells, and the like.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

"Plurality of sites" refers to two or more sites on a scaffold molecule capable of binding two or more compounds per scaffold molecule. Depending upon the nature of the scaffold and the compounds, 100 or more, 200 or more, or 300 or more compound molecules can be bound per scaffold molecule. In one aspect, the scaffold molecule is a protein of a viral nanoparticle, *e.g*., a CPMV nanoparticle.

"Terminal alkyne moiety" refers to an acetylenic bond (carbon-carbon triple bond) having a hydrogen attached to one carbon, e.g., R-C≡C-H, wherein R is a compound including, but not limited to, polynucleotide, polypeptide, glycopolymer, chromophoric dye, glycan, or lipid.

"Azide moiety" refers to a moiety, N≡N^{⊕}-N^{⊖}-. An azide moiety can be attached to a compound having a general structure, N≡N^{⊕}-N^{⊖}-R, wherein R is a compound including, but not limited to, polynucleotide, polypeptide, glycopolymer, chromophoric dye, glycan, or lipid.

The present invention provides an efficient strategy for end-functionalization of a compound, e.g., glycopolymer, polyethylene glycol, chromophoric dye, folic acid, glycan, lipid, polynucleotide, polypeptide, protein, or transferrin, using an azide-containing initiator for a living polymerization process followed by click chemistry elaboration of the unique azide end group. The copper-catalyzed cycloaddition reaction provides very efficient coupling of such polymers to a functionalized viral coat protein with efficient use of coupling reagents, compound molecules, and scaffold molecules. In an embodiment of the invention, a well-defined side chain neoglycopolymer possessing a single activated chain end can be chemically conjugated efficiently to a protein or bionanoparticle in a "bioorthogonal" fashion. The bioorthogonal labeling of biomolecules provides a unique, *in vivo* label that is an important tool for the study of biomolecule function and cellular fate. Attention is increasingly focused on labeling of biomolecules in living cells, since cell lysis introduces many artefacts. The method further provides high diversity in the nature of the label used in the ligation reaction.

In one embodiment, the method for coupling a compound to a scaffold comprises catalyzing a reaction between a first reactant having a terminal alkyne moiety and second reactant having an azide moiety for forming a product having a triazole moiety by addition of a metal ion in the presence of a ligand. The metal ion includes, but is not limited to, Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg. In a detailed embodiment, the metal includes, but is not limited to, Mn, Fe, Co, Cu, Mo, Tc, Ru, Rh, Pd, W, Re, Os, Ir, Pt, or Au. See for example, PCT International Application WO 2003/101972.

In a further detailed embodiment, the metal is heterogeneous copper, metallic copper, copper oxide, or copper salts.

Copper(I) salts, for example, Cu(I), CuOTf●C₆H₆ and [Cu(NCCH₃)₄]PF₆, can also be used directly in the absence of a reducing agent. These reactions usually require acetonitrile as co-solvent and one equivalent of a nitrogen base (*e.g*., 2,6-lutidine, triethylamine, diisopropylethylamine, or pyridine). However, formation of undesired byproducts, primarily diacetylenes, bis-triazoles, and 5-hydroxytriazoles, was often observed. For a recent summary of the reactions of Cu(I) complexes with dioxygen, see Schindler, Eur. J. Inorg. Chem. 2311-2326, 2000 and Blackman and Tolman in Structure and Bonding, B. Meunier, Ed., Springer-Verlag, Berlin, Heidelberg, 97: 179-211, 2000. This complication with direct use of Cu(I) species was minimized when 2,6-lutidine was used, and exclusion of oxygen further improved product purity and yield.

In one embodiment, the ligation reaction can be catalyzed by addition of Cu(I). If Cu(I) salt is used directly, no reducing agent is necessary, but acetonitrile or one of the other ligands indicate above can be used as a solvent (to prevent rapid oxidation of Cu(I) to Cu(II) and one equivalent of an amine can be added to accelerate the reaction. In this case, for better yields and product purity, oxygen should be excluded. Therefore, the ascorbate or any other reducing procedure is often preferred over the unreduced procedure. The use of a reducing agent is procedurally simple, and furnishes triazole products in excellent yields and of high purity. Addition of an amine, such as triethylamine or 2,6-lutidine to the acetonitrile system, solves the problem of reactivity ― the product is formed in quantitative yield after approximately 8 hours.

In a further embodiment, the ligation reaction can be catalyzed by addition of Cu(II) in the presence of a reducing agent for reducing the Cu(II) to Cu(I), *in situ.* Cu(II) salts, *e.g*., CuSO₄●5H₂O, can be less costly and often purer than Cu(I) salts. Reducing agents useful in this reaction include, but are not limited to ascorbic acid, sodium ascorbate, quinone, hydroquinone, vitamin K1, glutathione, cysteine, Fe²⁺, Co²⁺, and an applied electric potential. See, for example, Davies, Polyhedron 11: 285-321 1992, and Creutz, Inorg. Chem. 20: 4449, 1981. In a further example, metals can be employed as reducing agents to maintain the oxidation state of the Cu (I) catalyst or of other metal catalysts. Metallic reducing agents include, but are not limited to, Cu, Al, Be, Co, Cr, Fe, Mg, Mn, Ni, and Zn. Alternatively, an applied electric potential can be employed to maintain the oxidation state of the catalyst.

In a further embodiment, the ligation reaction can be catalyzed by addition of Cu(0) in the presence of an oxidizing agent for oxidizing the Cu(0) to Cu(I), *in situ.* Metallic containers can also be used as a source of the catalytic species to catalyze the ligation reaction. For example, a copper container, Cu(0), can be employed to catalyzed the ligation reaction. In order to supply the necessary ions, the reaction solution must make physical contact with the a copper surface of the container. Alternatively, the reaction can be run in a non-metallic container, and the catalytic metal ions supplied by contacting the reaction solution with a copper wire, copper shavings, or other structures. Although these reactions may take longer to proceed to completion, the experimental procedure reduces the number of intervening steps.

In one embodiment, the method for coupling a compound to a scaffold comprises catalyzing a reaction between a first reactant having a terminal alkyne moiety and second reactant having an azide moiety for forming a product having a triazole moiety by addition of a metal ion in the presence of a ligand for the metal ion. The metal ion is coordinated to a ligand for solubilizing such metal ion within the solvent, for inhibiting oxidation of such metal ion, and for dissociating, in whole or in part, from such metal ion during the catalysis of the reaction. Ligands can be, for example, monodentate ligands,bidentate (chelating) ligands, or multidentate ligands. Monodentate ligands refers to Lewis bases that donate a single pair ("mono") of electrons to a metal atom. Monodentate ligands can be either ions (usually anions) or neutral molecules. Monodentate ligands include, but are not limited to, fluoride ion (F⁻), chloride ion (Cl⁻), bromide ion, (Br⁻), iodide ion (I⁻), water (H₂O), ammonia (NH₃), hydroxide ion (OH⁻), carbon monoxide (CO), cyanide (CN⁻), or thiocyanate ion (CN-S⁻).

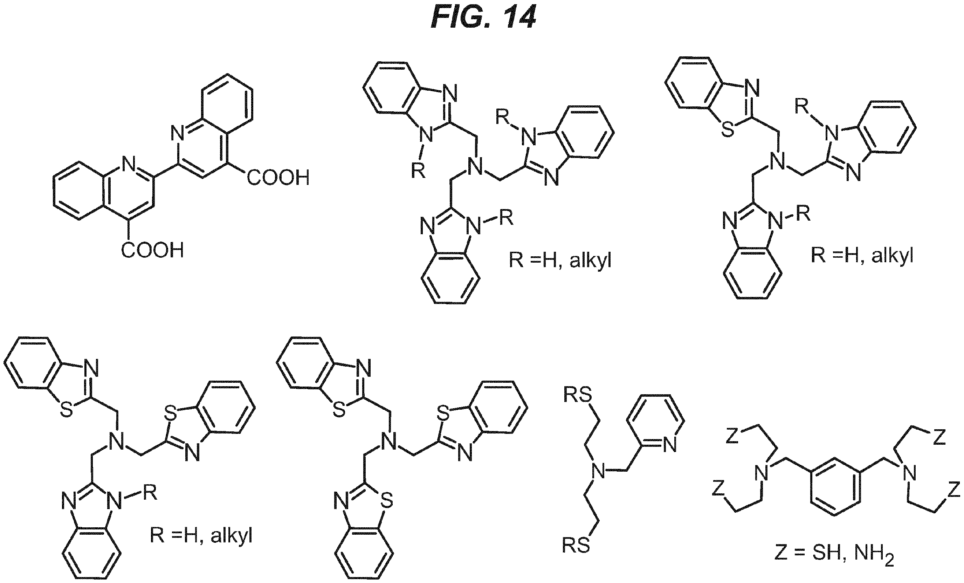

Bidentate ligands or chelating ligands refers to Lewis bases that donate two pairs of electrons to a metal atom. Bidentate ligands include, but are not limited to, ethylenediamine, acetylacetonate ion, phenanthroline, sulfonated bathophenanthroline or oxalate ion. Further examples of bidentate or chelating ligands are shown in Figure 14.

Ligands include, but are not limited to, acetonitrile, cyanide, nitrile, isonitrile, water, primary, secondary or tertiary amine, a nitrogen bearing heterocycle carboxylate, halide, alcohol, and thiol sulfide, phosphine, and phosphite. In a detailed embodiment, the halide is chloride and can be used at a concentration of 1-5 M. Polyvalent ligands that include one or more functional groups selected from nitrile, isonitrile, primary, secondary, or tertiary amine, a nitrogen bearing heterocycle, carboxylate, halide, alcohol, thiol, sulfide, phosphine, and phosphite can also be employed.

The ligation reactions as provided herein are useful for in a method for coupling a compound to a scaffold. The method provides catalyzing a ligation reaction between one or more terminal alkyne moieties and one or more azide moieties, for forming a product having a triazole moiety, the ligation reaction being catalyzed by addition of a metal ion in the presence of a ligand, and the scaffold having polyvalent sites for coupling to one or more compounds. In one aspect, the one or more terminal alkyne moieties are attached to the compound, and the one or more azide moieties are attached to the scaffold. In a further aspect, the one or more terminal alkyne moieties are attached to the scaffold, and the one or more azide moieties are attached to the compound. In a detailed aspect, the scaffold can be a protein on a viral nanoparticle, for example, a cow pea mosaic viral nanoparticle.

### EXEMPLARY EMBODIMENTS

### EXAMPLE 1

### Polyvalently Displayed Carbohydrates on Viral Nanoparticles

The strength and selectivity of binding interactions between polyvalently displayed carbohydrates and target cells are likely to depend on the number and flexibility of the arrayed sugars. In one aspect of the invention, a virion can be covered as densely as possible with carbohydrate groups. Increasing the degree of virus coverage requires the reactive polymer end group to be compatible with polymer synthesis and/or elaboration and yet reactive enough to accomplish a demanding subsequent connection to the virus coat protein ― a union of two large molecules present in low concentrations.

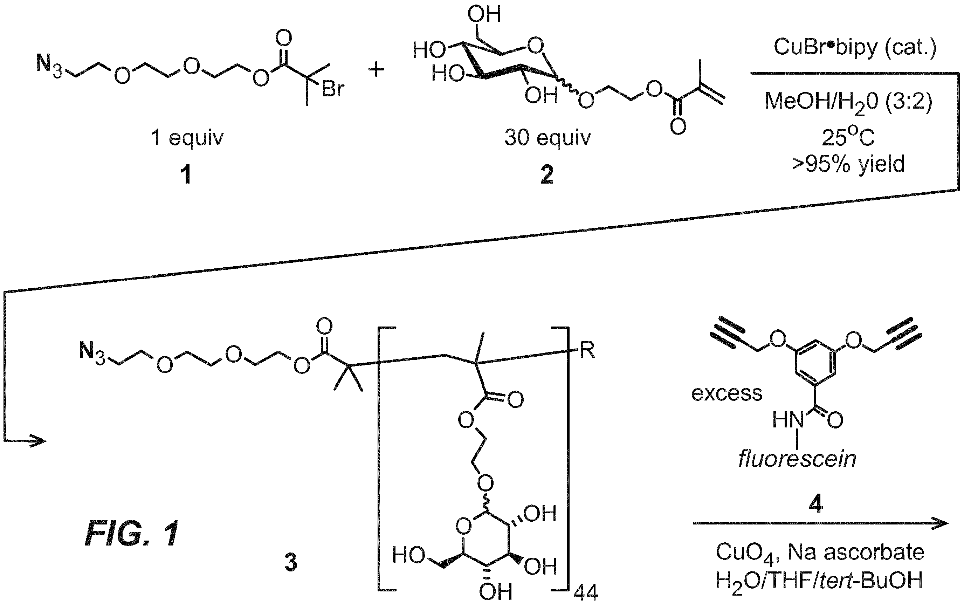

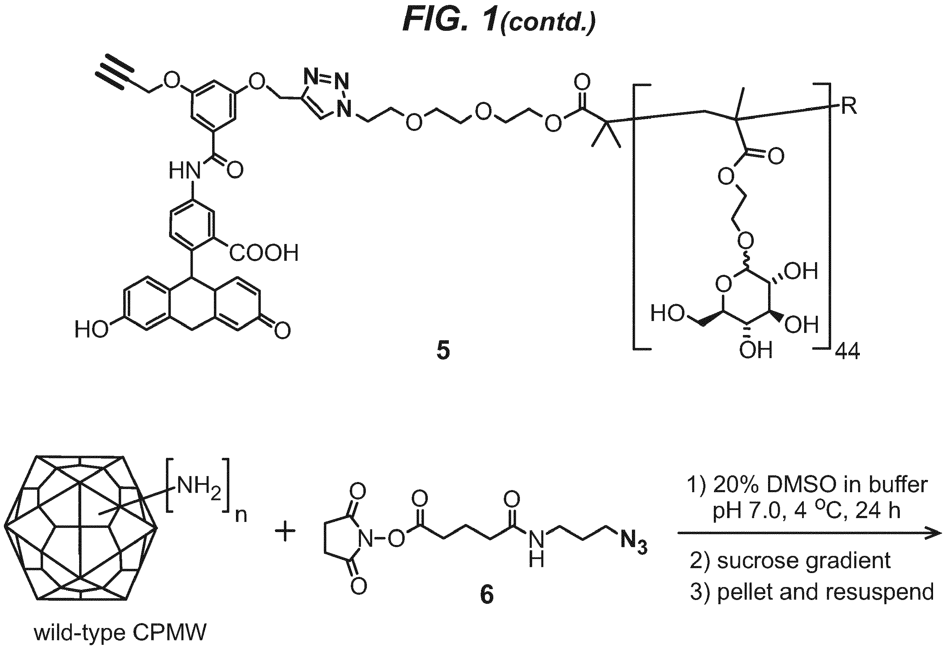

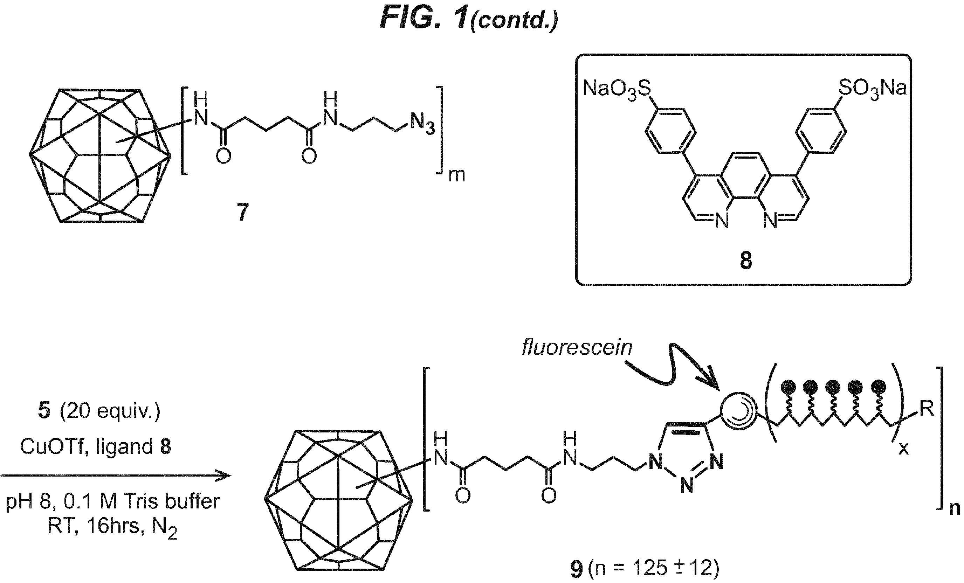

The side-chain neoglycopolymer **3** was prepared by atom transfer radical polymerization (ATRP) of methacryloxyethyl glucoside **(2)** using azide-containing initiator **1** (Figure 1). Gaynor et al., Macromolecules 31: 5951, 1998; Narain and Armes, Macromolecules 36: 4675, 2003. The presence of the azide chain end in the polymer was confirmed by colorimetric test and by the presence of the characteristic peak at 2100 cm―1 in the infrared spectrum. Punna and Finn, Synlett, 99, 2004. GPC analysis established the clean nature of the material and an average molecular weight (Mn) of 13,000 with polydispersity of 1.3, consistent with the initiator: monomer ratio used and with expectations for ATRP of acrylates in water. Narain and Armes, Macromolecules 36, 4675, 2003; Matyjaszewski, Chem. Eur. J. 5: 3095, 1999; Coessens and Matyjaszewski, J. Macromol. Sci.-Pure Appl. Chem. A36: 667, 1999; Li et al., J. Polym. Sci. A: Polym. Chem. 38: 4519, 2000.

Azide-terminated polymer **3** was elaborated to the alkyne-terminated form **5** by reaction with fluorescein dialkyne **4.** Figure 1. The excess dye was removed by filtration and the polymer products were further purified by size-exclusion chromatography (Sephadex G-15). The complete conversion of the azide to the alkyne end group was confirmed by the observation of a negative colorimetric test and by the disappearance of the azide IR resonance (the corresponding alkyne resonance is much less intense and therefore not visible). The chromophore thus installed serves as a spectroscopic reporter for subsequent manipulations. The dimeric polymer, formed as a minor byproduct from the reaction of two molecules of **3** and one of **4,** was not separated from **5** as it cannot participate in bioconjugation.

Cow pea mosaic virus (CPMV) was derivatized with N-hydroxysuccinimide **6** (NHS) to install azide groups at lysine side chains of the coat protein. Figure 1. NHS esters have been previously established to acylate lysine residues over the external surface of the capsid, with loadings controlled by overall concentration, pH, and reaction time. Wang et al., Chem. Biol. 9: 805, 2002. In this case, conditions were employed which result in the derivatization of a substantial fraction of the approximately 240 solvent-accessible lysine side chains (m = approximately 150 in Figure 1). The resulting azide-labeled virus **(7)** was then condensed with 20 equivalents of polymer-alkyne **5** in the presence of copper(I) triflate and sulfonated bathophenanthroline ligand **8** under inert atmosphere to produce the glycopolymer-virus conjugate **9** in excellent yield after purification by sucrose-gradient sedimentation to remove unattached polymer. Lewis et al., J. Am. Chem. Soc. 126: 9152, 2004. By virtue of the calibrated dye absorbance, the number of covalently bound polymer chains was found to be 125±12 per particle, representing the addition of approximately 1.6 million daltons of mass to the 5.6 million Da virion. This procedure, the general application of which will be described elsewhere, is far more efficient than the previous Cu(I)-mediated method, which required 100 equivalents of **5** with respect to azide to achieve similar results. Wang et al., J. Am. Chem. Soc. 125:3192,2003.

### EXAMPLE 2

### Covalent Labeling of CPMV Protein Subunits with Glycopolymer

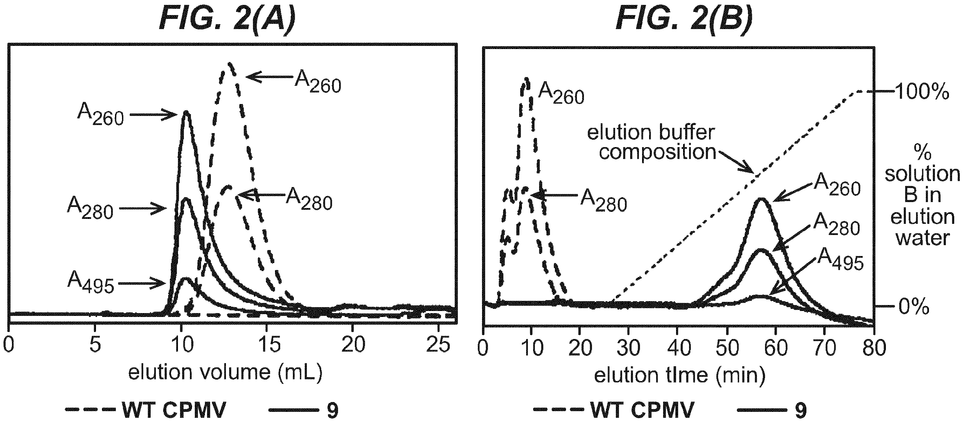

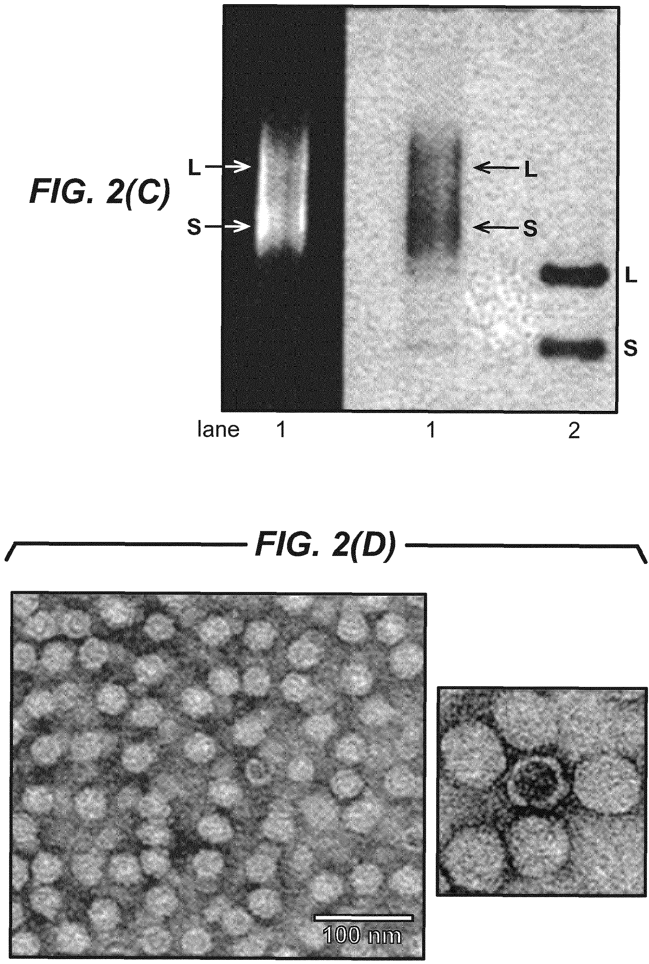

Covalent labeling of the vast majority of CPMV protein subunits with glycopolymer was confirmed by denaturing gel electrophoresis (Figure 2C). The intact nature of the particle assembly and its larger size was verified by size-exclusion FPLC (Figure 2A) as well as transmission electron microscopy (TEM, Figure 2D). TEM images revealed the virus conjugates to be more rounded in shape, to take on uranyl acetate stain differently, and to be 12-15% larger in diameter than the wild-type particle. The hydrodynamic radius and molecular weight of **9** were found by multi-angle dynamic light scattering (DLS) to be dramatically larger as well: 30.3 ± 3.4 nm and 1.4 ± 0.4 x 10⁷ Da, compared to 13.4 ± 1.3 nm and 6.1 ± 0.3 x 10⁶ Da for wild-type CPMV. That both radius and molecular weight values are substantially greater than expected reflects the uncertainties of calibration and interpretation of light scattering data for these unique polymer-virus hybrid species.

The glycosylated particles interacted strongly with both an immobilized form of the glucose-binding protein concanavalin A (Figure 2B) and with tetrameric conA in solution. The latter process resulted in the formation of large aggregates, the rate of which was monitored by light scattering at 490 nm. At a concentration of 0.7 mg/mL in **9** (approximately 0.1 µM in virions) and 0.3 mg/mL in conA, aggregation occurred within seconds, as expected for the efficient formation of a network by a large and polyvalent particle. See Examples 4 and 5.

Figure 2 shows (A) Size-exclusion FPLC (Superose 6) of wild-type CPMV and glycopolymer conjugate **9**. Protein from disassembled particles would appear at longer retention times than the peaks observed here, and the A₂₆₀/A₂₈₀ ratios are characteristic of intact, RNA-containing capsids for both samples. The more rapid elution of **9** is indicative of a substantial increase in the size of the particle, as 10 mL is the void volume of the column. Dye absorbance at 495 nm appears only for **9**. (B) FPLC on concanavalin-A Sepharose column of wild-type CPMV and virus-polymer conjugate **9**. The elution buffer was the indicated gradient mixture of 20 mM Tris-HCl, pH 7.4, with 0.15 M NaCl, 0.1mM Ca²⁺, and 0.1mM Mn²⁺ (solution A) and 1M glucose (solution B). (C) SDS-PAGE of **9** (lane 1) and WT-CPMV (lane 2). On the right (light background) is the gel visualized after Coumassie blue staining; note that almost all of the protein is converted to a slower-eluting form, expected for protein-glycopolymer conjugation. On the left (dark background) is the gel illuminated by ultraviolet light before staining (lane 2 shows no emission and is omitted). The arrows mark the center of the bands derived from the small (S) and large (L) subunits; their broad nature derives from the polydispersity of the polymer and the possibility for more than one attachment of polymer per protein subunit. (D) (*Left*) Negative-stained TEM of **9.** (*Right*) Enlarged TEM image of a WT-CPMV particle surrounded by **9.**

The present invention has demonstrated an efficient strategy for end-functionalization of glycopolymers, using an azide-containing initiator for a living polymerization process followed by click chemistry elaboration of the unique azide end group. Azide-alkyne cycloaddition with a chromophoric dialkyne served to label the polymer with a single dye molecule, allowing for convenient monitoring of further manipulations. The copper-catalyzed cycloaddition reaction provides very efficient coupling of such polymers to a functionalized viral coat protein. This method outperforms bioconjugation procedures previously used for polymer attachment to proteins such as acylation of lysine amine groups by activated esters and reaction of cysteine thiols with 2-thiopyridyl disulfides. To the best of our knowledge, this is the first time a well-defined side chain neoglycopolymer possessing a single activated chain end has been chemically conjugated to a protein or bionanoparticle in such a "bioorthogonal" fashion.

Particles such as **9** have extraordinarily high binding affinities for lectins in the canonical hemaglutinnation assay. ATRP/AAC methodology is being used to synthesize a range of glycopolymer-CPMV conjugates targeted toward overexpressed carbohydrate receptors in cancer cells.

### EXAMPLE 3

### Fluorophore-Labeled Glycopolymer Chains on a Virus Particle Scaffold

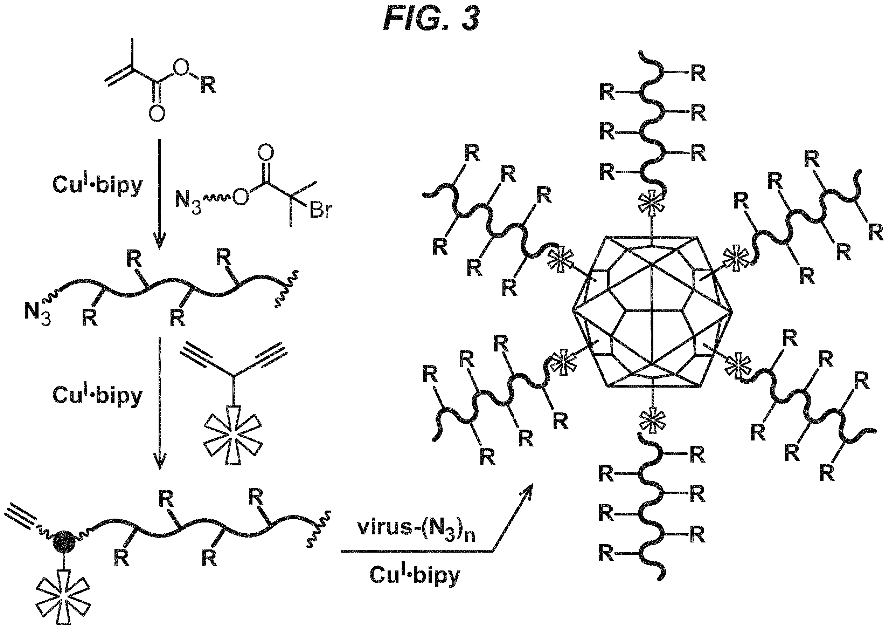

The construction of polymer-covered surfaces is made convenient by Cu(I) catalysis of polymerization, end-labeling, and attachment steps. The example described here is fluorophore-labeled glycopolymer chains on a virus particle scaffold. See Figure 3.

### EXAMPLE 4

### General Procedure for Modification of CPMV with Chemical Reagents.

Organic reagents were introduced into a solution of virus, such that the final solvent mixture was composed of 80% buffer and 20% DMSO. Unless otherwise specified, "buffer" refers to 0.1 M phosphate, pH 7.0. Purification of larger quantities of derivatized virus (>1 mg) was performed by ultracentrifugation over a 0-40% sucrose gradient, pelleting of the recovered virus, and solvation of the resulting material in buffer. Mass recoveries of derivatized viruses were typically 60-80%; all such samples were composed of>95% intact particles as determined by analytical size-exclusion FPLC. Virus concentrations were measured by absorbance at 260 nm; virus at 0.10 mg/mL gives a standard absorbance of 0.80. Fluorescein concentrations were obtained by measurement of absorbance at 495 nm, applying a calibrated extinction coefficient of 70,000. Each data point is the average of values obtained from three independent parallel reactions; loading values (the number of units attached to the virus) are subject to an experimental error of ±10%. The average molecular weight of the CPMV virion is 5.6 x 10⁶.

### EXAMPLE 5

### Syntheses

Synthesis of glycopolymers and virus-polymer conjugates in Figure 1. Compounds referred to in Examples 1 through 5 are in Figure 1.

*2-[2-(2-Azidoethoxy)ethoxy]ethanol:* A mixture of 2-[2-(2-chloroethoxy)ethoxy]ethanol (5.00 g, 29.7 mmol), sodium azide (9.6 g, 150 mmol) and a pinch of potassium iodide in water (50 mL) was stirred at 80 °C for 24 h. The reaction mixture was extracted with ether, and the organic solution was washed with brine and then dried over anhydrous Na₂SO₄. The solvent was evaporated and the product was dried under vacuum to give a colorless oil. ¹H NMR (CDCl₃, δ) 3.3-3.8 (m, 10H), 2.4 (m, 2H); ESI-MS *m*/*z* = 198.1 (M+Na); IR (KBr, cm⁻¹) 2100.

*2-Bromo-2-methylpropionic acid 2-[2-(2-Azidoethoxy)ethoxy]ethyl ester (**1**):* A solution of 2-bromoisobutyryl bromide (2.9 g, 12.6 mmol) and triethylamine (1.3 g, 12.8 mmol) in THF (20 mL) was cooled to 0 °C in a 3-necked round-bottomed flask. A solution of 2-[2-(2-azidoethoxy)ethoxy]ethanol (2.0 g, 11.4 mmol) in THF (20 mL) was added dropwise with stirring. The reaction mixture was then stirred at room temperature for 4 h, filtered, and the solvent was removed by rotatory evaporation. The crude product was added to a cooled (ice bath) 5% aqueous (Na₂CO₃) solution and the resulting mixture was extracted with ethyl acetate (3 x 100 mL). The combined organic layers were washed with water, dried over anhydrous (Na₂SO₄), and evaporated to provide **1** as a yellow oil. ¹H NMR (CDCl₃, δ) 4.2 (t, 2H), 3.4-3.8 (m, 8H), 3.2, (m, 2H), 1.9 (s, 6H), ESI-MS *m*/*z* = 346 (M+Na); IR (KBr, cm⁻¹) 2100.

*Poly(methacryloxy ethylglucoside) (**3**).* Methacryloxy ethylglucoside (2.48 g, 8.5 mmol), 2,2'-bipyridine (0.0882 g, 0.56 mmol), and **1** (0.091 g, 0.28 mmol) were dissolved in 3:2 methanol/water (20 mL) in a Shlenk flask. Nitrogen was bubbled vigorously through the mixture for 15 minutes and CuBr (0.0405 g, 0.282 mmol) was added. The mixture was maintained under a positive pressure of nitrogen at room temperature overnight. Exposing the reaction mixture to air stopped the polymerization. The methanol was removed under reduced pressure and 10 mL of water was added to the reaction mixture. Excess copper was removed using the commercial copper binding resin Cuprisorb™ and the resulting solution was washed with ethyl acetate (3 x 15 mL) to remove unreacted initiator and bipyridine. The resulting aqueous polymer solution was lyophilized overnight to afford a white flaky powder. The presence of the azide was confirmed by the modified ninhydrin test and by the presence of the azide peak in the IR spectrum (2100 cm⁻¹). Punna and Finn, Synlett 1: 99-100, 2004. ¹H NMR (D₂O, δ) 3.0-4.2 (m, 10H), 1.9 (m, 3 H), 0.7-1.1, (m, 2H). GPC was performed using polyethylene glycol and poly(*N,N*-dimethylacrylamide) calibration samples under standard conditions in water: Mₙ = 13,000, M_{w} = 10,000, polydispersity = 1.30.

*5-(3,5-Bis-prop-2-ynyloxy-benzoylamino)-2-(6-hydroxy-3-oxo-9,9a-dihydro-3H-xanthen-9-yl)-benzoic acid* (4). A mixture of fluorescein amine (1.53 g, 4.4 mmol) and sodium bicarbonate (0.8 g, 9.5 mmol) in dry THF (30 mL) was cooled in an ice bath and stirred under N₂ for 15 min. 3,5-Bis-prop-2-ynyloxy-benzoyl chloride (1.2 g, 4.84 mmol) in dry THF (40 mL) was added dropwise and the mixture was stirred overnight at room temperature. The solid bicarbonate was removed by filtration and the solvent was evaporated to give **4** as an orange solid, which was purified by column chromatography (silica gel, eluent 95:5 EtOAc:MeOH). ¹H NMR (CD₃OD, δ) 8.4 (s, 1H), 8.2 (d, 2H), 7.3 (m, 3H), 6.8-7 (m, 3H), 6.6-6.8 (m, 4H), 4.8 (d, 4H) (s, 6 H), 3.1 (t, 2H). ESI-MS *m*/*z* = 560.1 (MH⁺); UV-VIS (0.1 M phosphate, pH 7) λₘₐₓ 494 nm, ε = 64,000. Note that the reaction conditions used here, while convenient, may be adjusted to provide greater rates of cycloaddition by the use of a ligand for Cu(I). Lewis et al., J. Am. Chem. Soc. 126: 9152-9153, 2004.

*Polymer **5**.* A solution of **4** (120 mg, 0.214 mmol) in THF (2 mL) was added to a solution of 3 (107 mg, 0.0082 mmol) in H₂O (2 mL), followed by the addition of 2 mL *t*-BuOH. Sodium ascorbate (13 mg, 0.065 mmol) was added, followed by copper sulfate (8 mg, 0.032 mmol). The reaction mixture was capped (but not otherwise protected from oxygen) and stirred for 48 h at room temperature. The solvents were removed by rotary evaporation, water (10 mL) was added, and the most of the excess **4** was removed by extraction with ethyl acetate. The aqueous phase was concentrated by evaporation and the remaining residual **4** was removed by column chromatography over Sephadex G-15, eluting with water. The complete conversion of the azide to the alkyne end group was confirmed by the modified ninhydrin test and by the disappearance of the azide peak (2100 cm⁻¹) in the IR spectrum. ¹H NMR (D₂O, δ) 3.0-4.2 (m, 10H), 1.9 (3 H), 0.7-1.1, (2H); the aromatic end-group signals were not easily observed. Punna and Finn, Synlett 1: 99-100, 2004.

*5-(3-azidopropylamino)-5-oxopentanoic acid NHS ester* **6.** To a mixture of 5-(3-azidopropylamino)-5-oxopentanoic acid (410 mg, 1.9 mmol) and *N*-hydroxysuccinimide (242 mg, 2.1 mmol) in dry CH₂Cl₂ (25 mL) was added solid 1-ethyl-3-(3-dimethylaminopropyl)carbodiimide hydrochloride (EDC, 404 mg, 2.1 mmol) under nitrogen. The reaction was allowed to proceed for 12 hrs at room temperature. It was then washed with water (3 x 20 mL), dried over anhydrous Na₂SO₄, and the solvent was evaporated under reduced pressure to yield a white solid (417 mg, 70%). ¹H NMR (CDCl₃, δ) 6.2 (broad, NH),3.3-3.4 (m, 4H), 2.9 (s, 4H), 2.7 (t, 2H), 2.3 (t, 3H), 2.1 (m, 2H), 1.8 (m, 2H).

*Virus azide conjugate* **7.** Wild-type CPMV (24 mg, 0.25 µmol in protein asymmetric unit) was incubated with **6** (28.2 mg, 90 µmol) in 6 mL buffer containing 20% DMSO at RT for 12 hrs. The product was isolated by sucrose gradient sedimentation, ultracentrifugation pelleting, and resuspension in 0.1 M potassium phosphate buffer (pH 7.0), as previously described for similar reactions. Wang et al., Chem. Biol. 9: 805-811, 2002.

*Virus conjugate **9.*** Virus-azide **7** (4 mg, 7.1 x 10⁻⁴ µmol in viral capsids; approx. 0.11 µmol in azide) was incubated with **5** (140 mg, approx. 10.7 µmol) in a mixture of DMF (200 µL) and Tris buffer (pH 8, 0.1M, 1800 µL) in the presence of TCEP (4 mM), sulfonated bathophenanthroline ligand **8** (4 mM), and copper sulfate (2 mM) for 24 h at 4°C. The products were purified by two successive series of sucrose gradient sedimentation, ultracentrifugation pelleting, and resuspension in 0.1 M potassium phosphate buffer (pH 7.0). The materials were shown to be free of excess **5** by size-exclusion FPLC.

The use of ligand **10** ― the additive originally recommended and used for a variety of bioconjugation applications ― provides less efficient reactions in demanding, quantitative situations such as the present case. Chan et al., Org. Lett. 6**:** 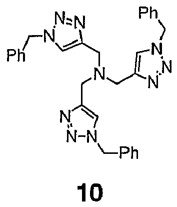 2853-2855, 2004; Link and Tirrell, J. Am. Chem. Soc. 125: 11164-11165, 2003; Link et al., J. Am. Chem. Soc. 126: 10598-10602, 2004. For example, the optimized use of **10** rather than sulfonated bathophenanthroline **8** requires the concomitant use of five times as much **5** to achieve a similar result, as follows. Virus-azide **7** (4 mg, 7.1 x 10⁻⁴ µmol in viral capsids; approx. 0.11 µmol in azide) was incubated with **5** (140 mg, approx. 10.7 µmol) in a mixture of DMF (200 µL) and Tris buffer (pH 8, 0.1M, 1800 µL) in the presence of tris(2-carboxyethyl)phosphine (4 mM), ligand **10** (4 mM), and copper sulfate (2 mM) for 24 h at 4°C. The product **9** was purified by two successive series of sucrose gradient sedimentation, ultracentrifugation pelleting, and resuspension in 0.1 M potassium phosphate buffer (pH 7.0). The same loading, but a slightly lower level of overall virus recovery, was observed.

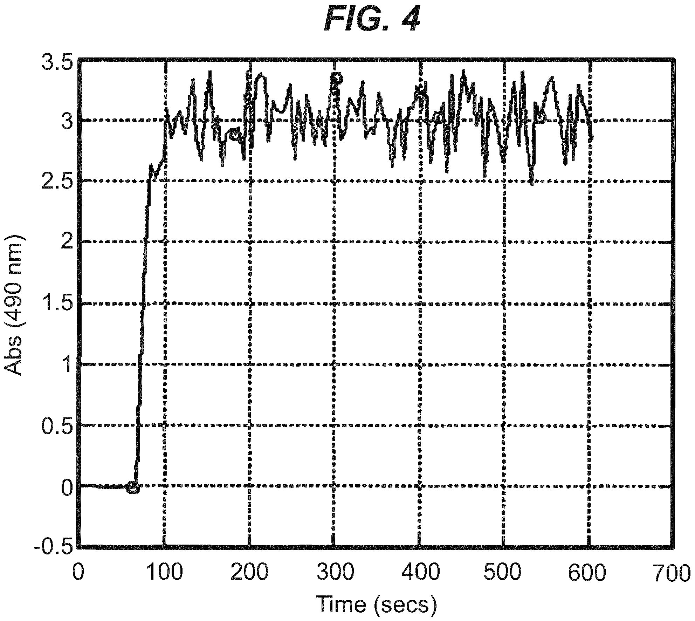

The rate of aggregation of **9** with conA was conveniently monitored at 490 nm, where absorbance of neither the icosahedral glycoprotein assembly nor con-A was observed (Figure 4). Figure 4 shows a time course of agglutination, monitored at 490 nm, for a mixture of con-A (0.32 mg/mL) and 9 (0.7 mg/mL) (26:1 molar ratio of con-A tetramer to virus particles, mixed at time 70 s) in PBS buffer with 0.1 mM Ca²⁺ and Mn²⁺.

### EXAMPLE 6

### Experimental Material

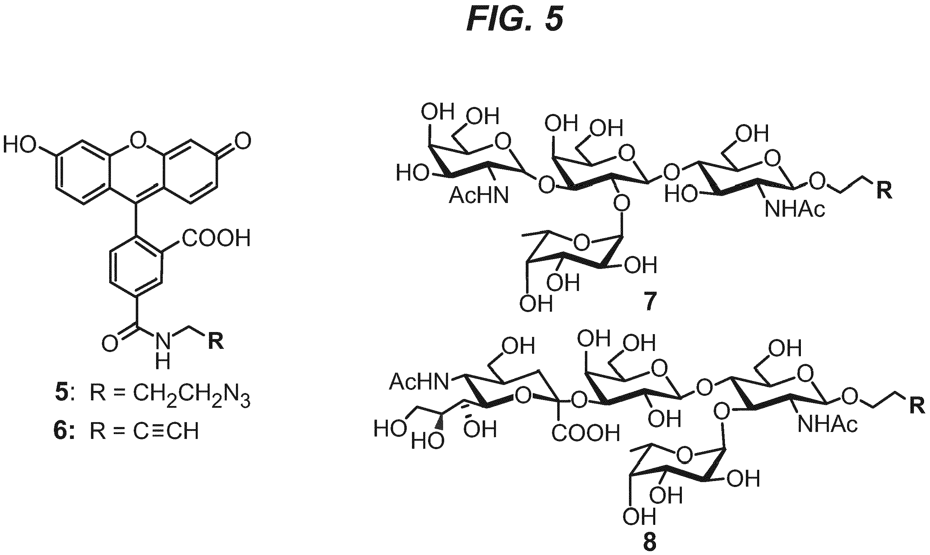

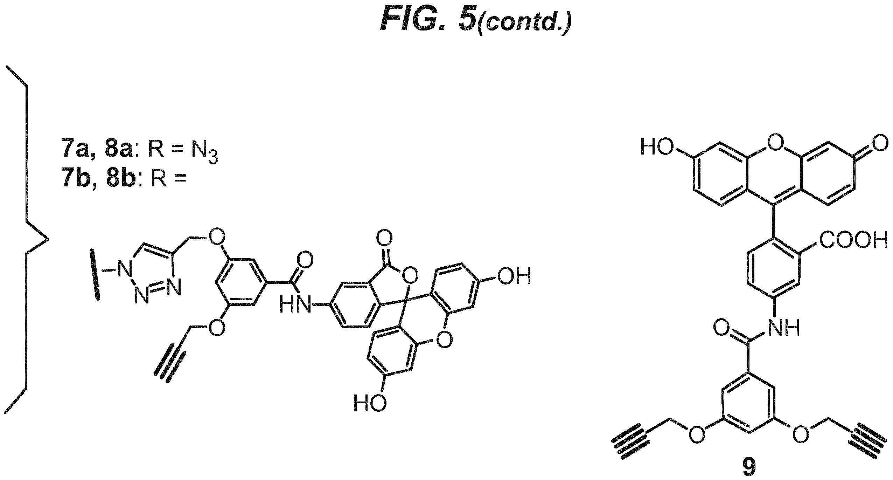

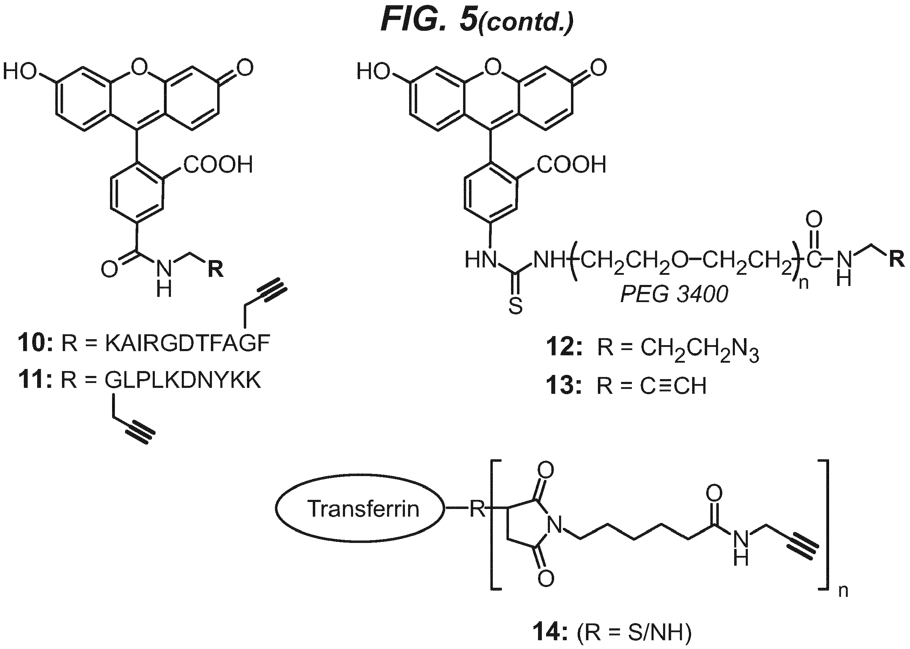

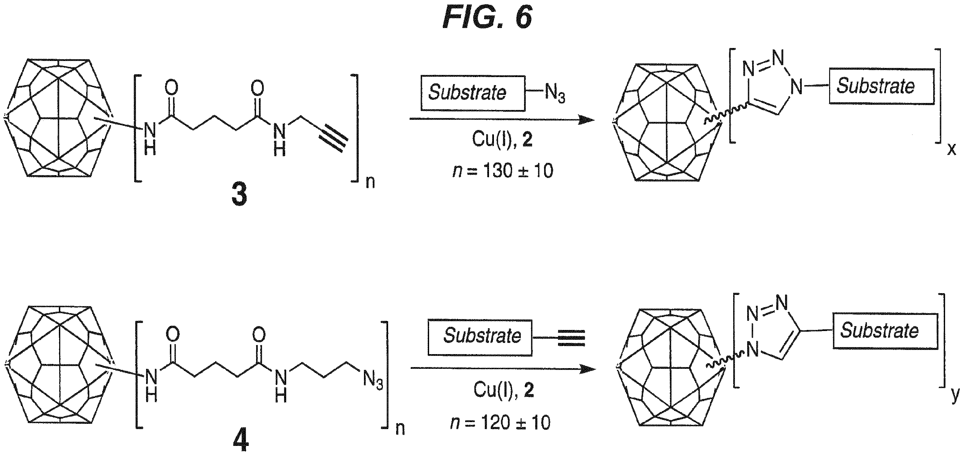

Substrates used and reaction scheme for Cu(I) mediated azide-alkyne cycloaddition (CuAAC) attachment to CPMV in Figures 5 and 6. Compounds referred to in Examples 6 through 13 are in Figures 5 and 6.

*Materials.* Fluorescein-PEG-NHS-3400 was obtained from Nektar (Huntsville, AL). Bathophenanthroline ligand **2** was purchased from GFS. Human holo-transferrin (98%) was supplied by Sigma. The resins Fmoc-Phe-Wang (0.77 mmol/g, 100-200 mesh) and Fmoc-Lys(Boc)-Wang (0.12 mmol/g, 100-200 mesh), as well as other Fmoc-protected amino acids were purchased from Chem-Impex International. Compounds **5, 6,** and [Cu(MeCN)₄](OTf) were prepared as previously described; **7a** and **8a** were provided by the Consortium for Functional Glycomics at The Scripps Research Institute. Wang et al., J. Am. Chem. Soc. 125: 3192-3193, 2003; Kubas, Inorg. Synth. 19: 90-92, 1979. CPMV-alkyne and -azide conjugates **3** and **4** were prepared as previously described using purified NHS esters of the acid-bearing linkers. Wang et al., J. Am. Chem. Soc. 125: 3192-3193, 2003. Fmoc-L-propargylglycine was purchased from CPSS (San Diego, CA). All other chemical reagents were obtained from commercial suppliers and used as received, unless indicated otherwise. Figures 5 and 6

*Instrumentation.* Air-sensitive manipulations were performed under nitrogen in a Vacuum Atmospheres glovebox. Preparative HPLC was performed with a Dynamax/Rainin Preppy SD-1 instrument and a Vydac protein and peptide reverse phase column, eluting with a gradient solvent mixture (solvent A = H₂O/0.1% TFA; solvent B = CH₃CN/0.1% TFA). MALDI-TOF analyses were performed by the Mass Spectrometry Facility at The Scripps Research Institute. FPLC analyses were performed on an AKTA Explorer (Amersham Pharmacia Biotech) equipped with a Superose-6 size exclusion column. Samples for TEM were obtained by deposition of 20 µL sample aliquots onto 100-mesh carbon-coated copper grids, followed by staining with 20 µL of 2% uranyl acetate. Images were obtained using a Philips CM100 electron microscope.

*Modification of CPMV with NHS Esters.* Reagents were introduced into a solution of CPMV, such that the final mixture contained ≤20% DMSO. Unless otherwise specified, the buffer used was 0.1 M phosphate, pH 7.0. Purification of derivatized virus (>1 mg) was performed by ultracentrifugation over a 10-40% sucrose gradient, pelleting of the recovered virus, and dissolution of the resulting material in Tris buffer (0.1 M, pH 8). Mass recoveries of derivatized viruses were typically 60-80%; all such samples were composed of >95% intact particles as determined by analytical size-exclusion FPLC. Virus concentrations were measured by absorbance at 260 nm; virus at 0.10 mg/mL gives a standard absorbance of 0.80. Fluorescein concentrations were obtained by measurement of absorbance at 495 nm, applying an extinction coefficient of 70,000 M⁻¹cm⁻¹. Each data point is the average of values obtained from three independent parallel reactions; loading values (the number of substrate molecules attached to the virus) are subject to an experimental error of ±10%. The average molecular weight of the CPMV virion is 5.6 x 10⁶ g/mole.

*Compounds **7b** and **8b.*** To a solution of **7a** (10 mg, 12.4 µmol) in H₂O (1 mL) was added 9 (70 mg, 0.125 mmol) in THF (1 mL). *t*-BuOH (1 mL) was added, followed by sodium ascorbate (0.5 M in H₂O, 72 µL, 36µmol) and CuSO₄ (0.5 M in H₂O, 24 µL, 12 µmol). The reaction mixture was stirred in a closed vial for 48 h at room temperature, followed by removal of the volatile solvents by rotary evaporation and addition of 5 mL H₂O. Excess 9 was largely removed by extraction with EtOAc. The reaction was monitored by TLC (R_{f} = 0.6 in 8:3:3:2 EtOAc/MeOH/AcOH/H₂O,) as well as by disappearance of the azide peak (2100 cm⁻¹) using FT-IR spectroscopy. The aqueous phase was concentrated by evaporation and residual **9** was removed by column chromatography (Sephadex G-15, 95:5 H₂O/BuOH), giving a yellow Solid (11 mg, 65% yield) upon lyophilization of the collected fraction. MALDI-TOF: [M+H]⁺ = 1361, [M+Na]⁺ = 1383, [M+K]⁺ =1399. Compound **8b** was synthesized in 55% yield from **8a** using the same procedure. MALDI-TOF: [M+Na]⁺ = 1472, [M+K]⁺ =1488.

*Compound **9.*** A mixture of fluorescein amine (1.57 g, 4.54 mmol) and sodium bicarbonate (1.57 g, 1.87 mmol) in dry THF (30 mL) was cooled in an ice bath and stirred under N₂ for 15 min. 3,5-Bis-prop-2-ynyloxy-benzoyl chloride (1.15 g, 4.99 mmol) in dry THF (30 mL) was added dropwise and the mixture was stirred overnight at room temperature. The solid bicarbonate was removed by filtration and the solvent was evaporated to give **4** as an orange solid, which was purified by column chromatography (silica gel, 95:5 EtOAc:MeOH). ¹H NMR (CD₃OD, δ) 8.4 (s, 1H), 8.2 (d, 2H), 7.3 (m, 3H), 6.8-7 (m, 3H), 6.6-6.8 (m, 4H), 4.8 (d, 4H) (s, 6 H), 3.1 (t, 2H). ESI-MS *m*/*z* = 560.1 (MH⁺); UV-vis (0.1 M phosphate, pH 7) λₘₐₓ 494 nm, *ε*= 64,000.

*Peptides **10** and **11***. Compound **10** was prepared by standard techniques of solid-phase Fmoc peptide synthesis using 0.2 mmol Fmoc-Phe-Wang resin. Coupling of Fmoc-L-propargylglycine was performed as reported elsewhere. Punna et al., Angew. Chem. Int. Ed. 44: 2005 in press. Conjugation of fluorescein to the N-terminus of the peptide chain was accomplished by addition of a DMF/*i*Pr₂NEt (2:1 v/v) solution containing 5(6)-carboxyfluorescein (414 mg, 1.1 mmol) and HBTU (417 mg, 1.1 mmol) to the drained resin. The mixture was agitated overnight and purified by reverse phase HPLC after cleavage from the resin. MALDI-TOF: [M+H]⁺ = 1579. Peptide **11** was obtained from the analogous procedure using 0.1 mmol Fmoc-Lys(Boc)-Wang resin. MALDI-TOF: [M+H]⁺ = 1571, [M+Na]⁺ = 1593.

*Polymer **12**.* A toluene solution of 3-azido-1-propylamine (0.66 M, 334 µL, 0.22 mmol) was added to a solution of fluorescein-PEG-NHS-3400 (150 mg, 0.044 mmol) in dry CH₂Cl₂ (5 mL). The reaction was stirred overnight, followed by removal of the solvents under reduced pressure. H₂O (10 mL) was added and the solution was extracted with EtOAc to remove the excess azide compound. The aqueous solution was lyophilized to afford a yellow powder (135 mg, 90% yield).

*Polymer **13.*** To a solution of fluorescein-PEG-NHS-3400 (150 mg, 0.044 mmol) in dry CH₂Cl₂ (5 mL) was added propargylamine (12.1 mg, 0.22 mmol). The reaction was stirred overnight and worked up as described for **12.** Compound **13** was isolated as a yellow powder (135 mg, 90% yield).

*Transferrin-Alkyne Conjugate **14.*** To human holo-transferrin (50 mg, 0.625 µmol) in phosphate buffer (0.1 M, pH 7, 2 mL) was added *N-(N-*(prop-2-ynyl)hexanamidyl)maleimide (3.9 mg, 9.1 µmol) in DMSO (500 µL), and the reaction was incubated overnight at room temperature. Purification through a G-15 Sephadex coloumn followed by dialysis and lyophilization afforded **14** as a pink powder (30 mg).

*Modification of CPMV by CuAAC Reaction.* CPMV conjugate **3** or **4** (1 mg as 2 mg/mL solution) was incubated with complementary azide or alkyne compound (concentrations given in Table 1) in Tris buffer (0.1 M, pH 8, 0.5 mL) containing **2** (3 mM) and [Cu(MeCN)₄](OTf) (1 mM) for 12 h at room temperature with rigorous exclusion of dioxygen. CPMV-**12**, CPMV-**13**, and CPMV-**14** conjugates were purified by sucrose gradients and pelleting as described above. All other CPMV conjugates were purified by size exclusion chromatography using Bio-Spin^{®} disposable chromatography columns filled with Bio-Gel^{®} P-100 as described elsewhere. Wang et al., Chem. Biol. 9: 805-811, 2002.

### EXAMPLE 7

### Optimization of Reaction Conditions

Sulfonated bathophenanthroline **2** is a highly efficient ligand in a fluorescence quenching catalysis assay prompted us to further investigate **2** for the coupling of compounds to suitably derivatized CPMV particles. Lewis et al., J. Am. Chem. Soc. 126: 9152-9153, 2004. The viral capsids were labeled with alkynes (**3**) or azides (**4**) at surface-exposed lysine residues using standard *N*-hydroxysuccinimide (NHS) ester chemistry (Figure 6). Wang et al., J. Am. Chem. Soc. 125: 3192-3193, 2003. Initial experiments were performed using functionalized fluorescein dyes as substrates, since the success of the bioconjugation could be readily monitored using UV-vis spectroscopy. Thus, fluorescein derivatives **5** and **6** (Figure 5) were condensed with 3 and 4, respectively, in the presence of Cu·**2** in Tris buffer (pH 8) under inert atmosphere, to give CPMV-dye conjugates with good loading in a concentration-dependent fashion. Wang et al., J. Am. Chem. Soc. 125: 3192-3193, 2003. In all cases, the reaction yield (the percent of virus recovered after purification of protein away from small molecules) and purity (intact virus particles vs. disassembled viral protein) was high. Thus, >85% of the protein was recovered in each case, and size-exclusion FPLC indicated that >95% of the virons were intact particles. See Supporting Information for details. SDS-PAGE analysis visualized under ultraviolet light revealed two dye-labeled bands corresponding to the small and large subunits of CPMV, indicating that both subunits of the virus were chemically modified. No attachment was found to occur in the absence of Cu(I), ruling out nonspecific adsorption of dye to virus. It should also be noted that the use of phosphate buffer diminishes the effectiveness of the reaction, while HEPES buffer is at least as good or better than Tris.

### EXAMPLE 8

### Dependence of Dye Loading on Reagent Concentration

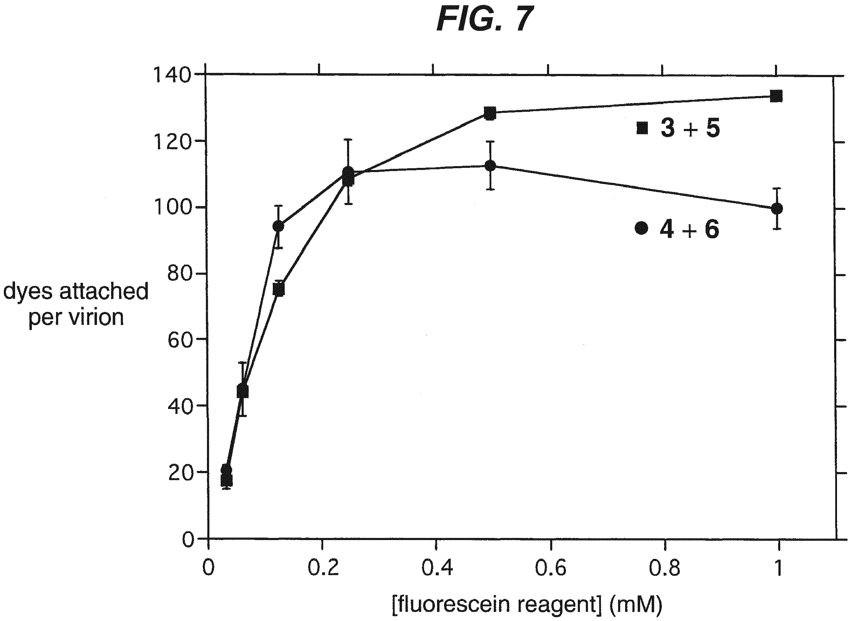

The dependence of the observed loading (dye attachments per virion) on substrate concentration is shown in Figure 7. Upon treatment of 2 mg/mL **4** (0.36 µM in virus particles) with 200 µM **6,** corresponding to a five-fold molar excess with respect to azide groups on **4,** the CPMV particles were found to be fully labeled (∼110 dyes/particle). Similar results were obtained with **3** + **5.** In contrast, the use of ligand **1** under otherwise identical conditions required a 5 mM concentration of **6** (250 equiv) to achieve such dye loadings. Furthermore, the reaction of opposite "polarity" (**3** + **5**) mediated by Cu·**1** was significantly worse than **4 + 6.** See Figures 5 and 6.

Figure 7 shows the dependence of dye loading on reagent concentration. Conditions used: 2 mg/mL **3** or **4,** complementary fluorescein derivatives **5** or **6,** 1 mM [Cu(MeCN)₄](OTf), 3 mM **2,** Tris-HCl buffer (pH 8), r.t., 14 hr.

The same results with each ligand were obtained using [Cu(MeCN)₄](OTf), [Cu(MeCN)₄](PF₆), or CuBr as the source of Cu^{I}. The optimal copper concentration was found to be 1 mM; lower concentrations significantly decreased the coupling efficiency. The ligand-to-metal ratio is also important. A 3:1 ratio of **2** to Cu^{I} afforded the best results; a lower ratio resulted in significant degradation of the viral capsid whereas a larger excess of ligand slowed the reaction to provide incomplete labeling. The efficiency of the Cu·**2** mediated AAC process thereby far exceeds that of standard NHS and maleimide coupling reactions with lysine and cysteine side chains, respectively. For example, the addition of a 10-fold excess of fluorescein NHS ester to CPMV under similar conditions results in the attachment of approximately 20 dyes to each capsid, and fluorescein maleimide deposits between 10 and 25 dye molecules on CPMV mutants bearing surface cysteine residues, depending on the local environment of the sulfhydryl groups. Wang et al., Chem. Biol. 9: 805-811, 2002; Strable and Finn, Unpublished work. While the linker used to attach azides and alkynes to CPMV may make these groups more accessible than the lysine or cysteine side chains of native and mutant forms of the particle, the differences should be small given the highly solvent-exposed nature of many of the surface peptide residues.

### EXAMPLE 9

### Preparation of CPMV-Carbohydrate Conjugates.

With the optimal reaction conditions thus established, biologically-relevant substrates were attached to the CPMV capsid (Figure 5; Table 1). Carbohydrate **7a** binds the protein galectin-4, an early marker of breast cancer cells. Blixt et al., Proc. Nat. Acad. Sci. USA 101: 17033-17038, 2004; Huflejt and Leffler, Glycoconj. J. 20: 247-255, 2004. Sialyl Lewis X, an azide derivative of which is **8a**, is overexpressed on cancer cells and also plays a role in inflammation. Ohyama et al., EMBO J. 18: 1516-1525, 1999. The attachment of these two compounds to the surface of a virus particle can be useful for drug targeting, as well as for the elusive goal of antibody production against carbohydrate epitopes. Seitz, ChemBioChem 1: 214-246, 2000. In order to allow for ready quantitation of the attachment of these non-fluorescent compounds, the azides were submitted to a CuAAC reaction with fluorescein dialkyne reagent **9** to provide dye-alkyne derivatives **7b** and **8b.** Using the Cu·**2** system, **7b** and **8b** were then successfully grafted to virus-azide **4** with loadings of 115 and 105 per virion, respectively. Only 4 equiv of **7b** or **8b** per azide group on **2** was necessary to reach this level of loading at a virus concentration of 1-2 mg/mL. The integrity of polyvalently-displayed **7** and the retention of the activity of the carbohydrate was verified by the formation of a gel upon the addition of CPMV-(**7b**)₁₁₅ to dimeric galectin-4. See Supporting Information for details. The use of **7a** and **8a** with particle **3** under similar conditions likewise gave intact derivatized virions in high yield with the ability to efficiently crosslink a solution of galectin-4. In these cases, the loading of small molecules lacking the fluorescein tag is approximately the same as for their fluorescent counterparts, since we have established with extensive studies that the nature of the substrate has little effect on the efficiency of the CuAAC reaction.

This facile attachment of complex, unprotected sugars to proteins by CuAAC ligation represents a significant advancement over existing methodologies employing a bifunctional linker on the carbohydrate for standard bioconjugation reactions. Typically, squarates and maleiimide-hydrazide or maleiimide-NHS ester linkers have been employed for this purpose, and the additional synthetic steps required to functionalize the sugars in the appropriate fashion result in poor overall coupling yields. Seitz, ChemBioChem 1: 214-246, 2000; *et al.,* Carb. Res. 313: 15-20, 1998; Hossany et al., Bioorg. Med. Chem. 12: 3743-3754, 2004; Allen et al., Chem. Eur. J. 6: 1366-1375, 2000. In contrast, azides can be readily incorporated into the carbohydrate scaffold early in the synthesis and rarely interfere in subsequent synthetic steps.

**Table 1. Azide-alkyne cycloaddition on CPMV (2 mg/mL; 47 µM in alkyne for 3, 43 µM in azide for 4) with various substrates.**

| Entry | Substrate | CPMV Derivative | [Substrate] (µM) | Loading | Yield (%) |
|---|---|---|---|---|---|
| 1 | **7b** | **4** | 200 | 115 | 85 |
| 2 | **8b** | **4** | 200 | 105 | 85 |
| 3 | **10** | **4** | 120 | 60 | 85 |
| 4 | **11** | **4** | 250 | 115 | 80 |
| 5 | **12** | **3** | 500 | 60 | 60 |
| 6 | **13** | **4** | 250 | 90 | 75 |
| 7 | **14** | **4** | 260 | — *^{a}* | — *^{a}* |

| | | | | | |
|---|---|---|---|---|---|
| (*a*) not determined | | | | | |

### EXAMPLE 10

### Attachment of Peptides to CPMV

Although the genetic incorporation of peptide loops into selected regions of the CPMV capsid structure is well established, the production of such chimeras suffers from restrictions in terms of size, position, and sequence. Taylor et al., Biol. Chem. 280: 387-392, 1999; Taylor et al., J. Mol. Recog. 13: 71-82, 2000; Chatterji et al., Intervirology 45: 362-370, 2002. Given the great importance of cyclic and linear peptides to a wide variety of targets in biochemistry, molecular recognition, and drug development, robust methods for the attachment of natural and non-natural oligopeptides to polyvalent scaffolds are of interest. To demonstrate the virtues of the CuAAC reaction in this regard, peptides were chosen containing carboxylic acid or amine side chain functional groups and which would therefore require protection/deprotection strategies to be incorporated in standard peptide coupling procedures. To date, the decoration of full proteins with functional peptides has been accomplished predominantly with native chemical ligation or maleimide-cysteine reactions. Dawson et al., Science 266: 776-779, 1994; Dawson and Kent, Ann. Rev. Biochem. 69: 923-960, 2000. Both of these strategies require the presence of accessible cysteine residues in the protein, the former at the N-terminus. Dibowski and Schmidtchen, Angew. Chem., Int. Ed. 37: 476-478, 1998.

The Cu·**2** system was tested with two functional peptides. The arginine-glycine-aspartate (RGD) sequence of **10** is derived from an adenovirus serotype that binds αᵥ integrins, extracellular matrix receptors that are overexpressed on many cancer cells. Nemerow and Stewart, Microbiol. Mol. Biol. Rev. 63: 725-734, 1999. The amino acid sequence of **11** comes from a portion of the protective antigen (PA) of anthrax toxin, a moiety that binds edema factor (EF) and lethal factor (LF) and permits cell entry of the toxin. Mogridge et al., Proc. Nat. Acad. Sci. USA 99: 7045-7048, 2002; Cunningham et al., Proc. Nat. Acad. Sci. USA 99: 7049-7053, 2002; Bradley et al., Nature 414: 225-229, 2001. Peptide **10** was successfully attached to **4** with a loading of 60 peptides per viral particle using only a 6 fold-excess of substrate and standard Cu·**2** conditions. Significantly, no peptide attachment was obtained when ligand **1** was employed with up to 5 mM substrate present. The attachment of **11** afforded a loading of 115 peptides/virion, and SDS-PAGE analysis by UV irradiation indicated that both small and large subunits of CPMV were modified with the PA peptide (data not shown). The ready incorporation of alkyne groups into synthetic peptides permits the Cu·**2**-mediated AAC reaction to serve as a general strategy for the attachment of peptides to biomolecular scaffolds. Punna et al., Angew. Chem. Int. Ed. 44: 2005 in press.

### EXAMPLE 11

### Preparation of Virus-Polymer Constructs

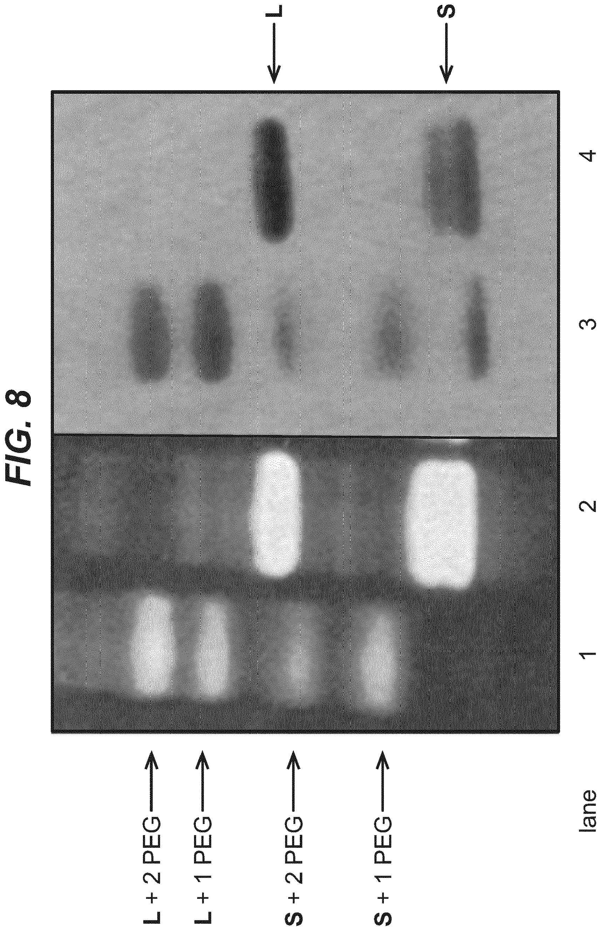

CPMV was previously derivatized with poly(ethylene oxide) (PEG) using an NHS ester derivative to give well-controlled loadings of the polymer on the outer coat-protein assembly. Raja et al., Biomacromolecules 4, 472-476, 2003. Compared with wild-type CPMV, the PEGylated particle showed altered physical properties and a reduced immunogenic response in mice. Lysine reactivity with PEG activated esters allowed one to reach a maximum of only 30 attached PEG molecules per virion. Attempts to boost the loading past this value required such a high concentration of PEG reagent that the virus particle precipitated before reaction could occur. However, the enhanced activity of the Cu·**2** catalyst now allows us to improve on this prior result. Thus, fluorescein end-functionalized PEG reagents **12** and **13** were coupled to their complementary CPMV alkyne and azide scaffolds to give loadings of 60 and 90 PEG chains per virion, respectively, using easily accessible concentrations in which the virus particles are stable (Table 1). The resulting particles were again less dense on sucrose gradient sedimentation and larger as indicated by size-exclusion FPLC. See Supporting Information for details. Figure 8 shows the denaturing gel of CPMV-(**13**)₉₀ and CPMV-(**5**)₁₁₀ visualized by UV irradiation and protein staining. In both cases, both large (L) and small (S) subunits of the CPMV coat protein were labeled, as expected. The PEG conjugate CPMV-**13** gives rise to two higher molecular weight bands for each subunit, corresponding to single and double labeling of the subunits by the polymer. Protein staining of this conjugate also reveals the presence of a small proportion of unmodified subunits.

Figure 8 shows SDS-PAGE of CPMV-(**13**)₉₀ (lane 1) and CPMV-(**5**)₁₁₀ (lane 2). On the right (light background) is shown the gel visualized after SimplyBlue™ staining; the two extra bands corresponding to each subunit arise from modification by 1 or 2 PEG-3400 moities. On the left (dark background) is the gel illuminated by UV light prior to protein staining. Because PEG-3400 is labeled with fluorescein, only the modified subunits are visible in lane 1. The two small-subunit bands appearing in lane 2 arise from incomplete C-terminal peptide cleavage *in vivo* and are unrelated to the present experiments. Taylor et al., Virology 255: 129-137, 1999.

### EXAMPLE 12

### Attachment of the Transferrin Protein

As a final example of the ability of Cu·**2** to efficiently promote the AAC reaction, the coupling of a large protein to the outer surface of CPMV was performed. Receptors for transferrin (Tfn), an iron carrier protein in vertebrates, are overexpressed on a variety of cancer cells. Polyvalent assemblies of Tfn on such scaffolds as liposomes and iron oxide nanoparticles have therefore been prepared for cancer cell targeting. Hogemann-Savellano et al., Neoplasia 5: 495-506, 2003; Ryschich et al., Eur. J. Cancer 40: 1418-1422, 2004; Derycke et al., J. Nat. Cancer Inst. 96: 1620-1630, 2004. The display of multiple copies of Tfn on CPMV could similarly afford a particle that binds tightly and selectively to receptor-bearing cells.

Human holo-transferrin, an 80 kDa bilobed glycoprotein, was incubated at high concentration (20 mg/mL) with 15 equiv. of a maleimide-alkyne linker at pH 7 to afford the alkyne-derivatized protein **14,** with attachments made at one or more accessible cysteines (and perhaps, to a lesser extent, lysine) residues. The successful conjugation of alkyne to Tfn was verified by reaction of **14** with the fluorescein derivative **5** under CuAAC conditions. Analysis by SDS-PAGE confirmed that all of the Tfn was covalently labeled with at least one fluorescein molecule (data not shown).

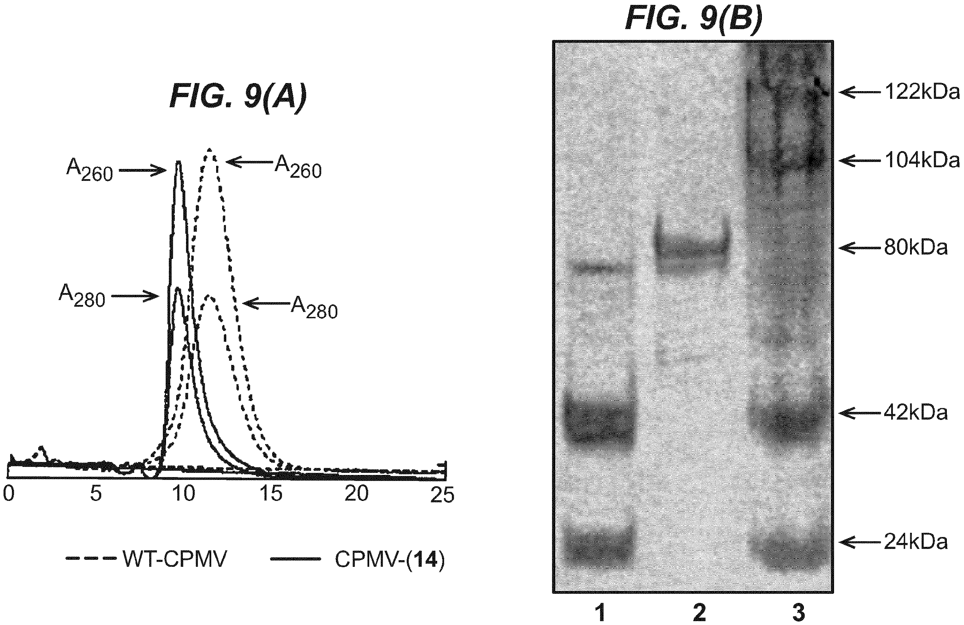

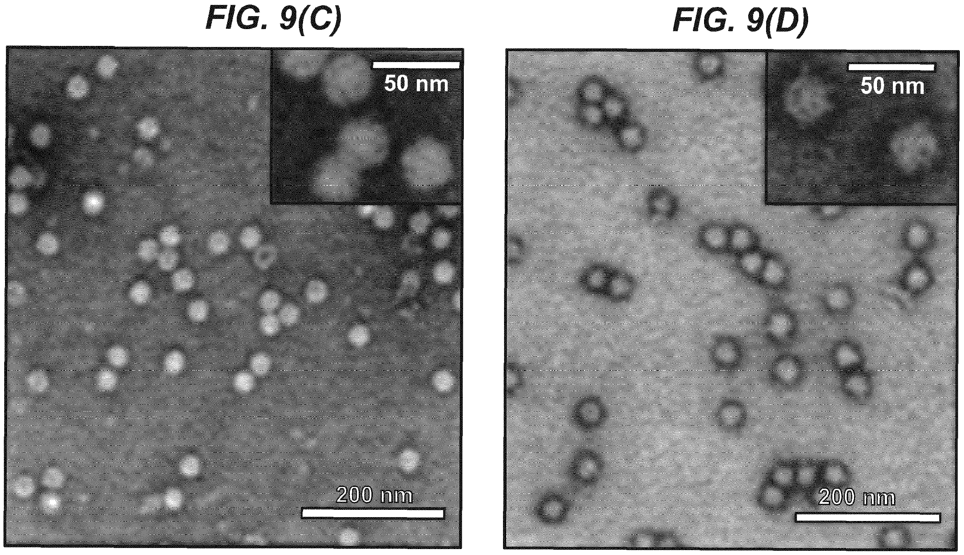

The CPMV-Tfn conjugate CPMV-(**14**)ₙ was then prepared by reaction of **4** with **14** using Cu·**2**. Examination of the product by FPLC, SDS-PAGE, TEM (Figure 9) and Western immunoblotting indicated that a significant number of Tfn molecules were arrayed on the particle. See Supporting Information for details. Importantly, the virus-protein conjugates were isolated as individual particles, with no evidence of aggregation that might be expected if Tfn species bearing more than one alkyne were to couple to polyvalent CPMV azides. In negative-stained electron microscopy, individual CPMV-(**14**)ₙ particles were larger than wild-type CPMV by approximately 16 nm in diameter, and displayed a clear knobby appearance contrasting with the smooth hexagonal shape of the wild-type virion. These observations confirm that Tfn molecules were covalently attached evenly over the CPMV surface. Preliminary measurements show the attached Tfn molecules to be active in binding the target receptor.

A recent report employing thiol-maleimide chemistry for the attachment of proteins (up to 22 kD) to CPMV required the use of a 50-fold excess of protein with respect to viral asymmetric unit, as well as subsequent chromatographic purification of the desired conjugate. Chatterji et al., Bioconj. Chem. 15: 807-813, 2004. We have obtained many similar results for NHS ester-lysine as well as thiol-electrophile reactions. In contrast, protein conjugation is achieved by the CuAAC reaction with efficiencies comparable to those of native chemical ligation (NCL). Dawson et al., Science 266: 776-779, 1994; Dawson and Kent, Ann. Rev. Biochem. 69: 923-960, 2000. Here, only a 6-fold excess of Tfn was required, and the relatively small amount of Tfn employed allowed for simple purification by sucrose gradients and pelleting. While NCL reactions are typically conducted with nearly equimolar ratios of coupling reagents, the concentrations of thioester and *N*-terminal cysteine reaction partners are typically much higher (0.1-1 mM) than the CPMV azides and alkynes used here. Dawson et al., J. Am. Chem. Soc. 119: 4325-4329, 1997; Xu et al., Proc. Nat. Acad. Sci. USA 96: 388-393, 1999; Offer et al., J. Am. Chem. Soc. 124: 4642-4646, 2002; Bang and Kent, Proc. Nat. Acad. Sci. USA 102: 5014-5019, 2005. The Cu·**2**-mediated AAC protocol is therefore an excellent alternative for the coupling of suitably functionalized proteins. Bausinger et al., ChemBioChem 6: 625-628, 2005.

An embodiment of the present invention provides a highly efficient azide-alkyne cycloaddition protocol using a simple copper(I) salt and sulfonated bathophenanthroline (**2**) for chemoselective ligation. This catalytic system permits the attachment of complex carbohydrates, peptides, polymers, and proteins to biomacromolecules in yields and substrate loadings far superior to those possible with previously established procedures. Advantages to the Cu·**2-**mediated AAC method include the use of modest excesses of the desired coupling partners and simple purification. The unfortunate tendency of copper ions to speed the hydrolytic cleavage of peptides and polynucleotides is largely controlled by the use of enough ligand to restrict access to the metal center. The improved CuAAC reaction can be particularly beneficial to those wishing to join substrates that are expensive or available in only small quantities, and for biological molecules in which azides or alkynes are incorporated by biosynthetic procedures.³³ The single drawback to this system is the requirement that the reaction be performed under inert atmosphere; ligands designed to solve this problem are currently being developed.

Figure 9 shows (A) Size-exclusion FPLC of wild-type CPMV and CPMV-(**14**)ₙ. Protein from disassembled particles would appear at longer retention times than the peaks observed here, and the A₂₆₀/A₂₈₀ ratios are characteristic of intact, RNA-containing capsids for both samples. The more rapid elution of CPMV-(**14**)ₙ indicates a substantial size increase in the particle, as 10 mL is approximately the void volume of the column. (B) SimplyBlue™-stained gel (4-12% bis-tris) of wild-type CPMV (subunits at 42 and 24 kDa) (lane 1), Tfn (80 kDa) (lane 2) and CPMV-(**14**)ₙ (lane 3). Note the appearance of two strong bands of approximately 102 and 122 kDa in the lane 3, corresponding to the CPMV subunits conjugated with Tfn. (C) Negative-stained TEM of wild-type CPMV. (D) Negative-stained TEM of CPMV-(**14**)ₙ. Automated measurement of the particles showed the average diameters to be 30±1 nm for wild-type and 46±5 nm for CPMV-(**14**)ₙ.

### EXAMPLE 13

### Characterization of CPMV conjugates

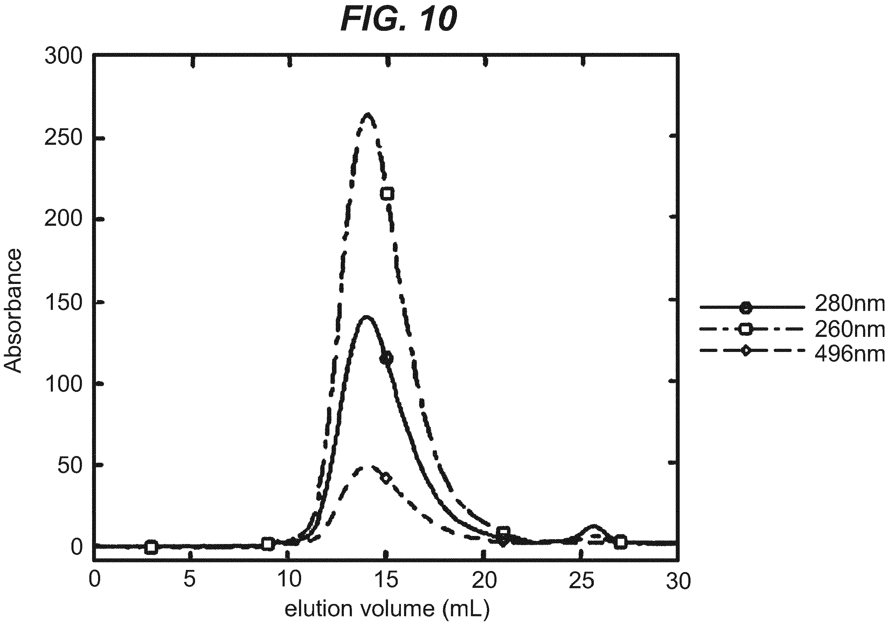

All CPMV conjugates were characterized by analytical size exclusion FPLC. The representative trace shown in Figure 10 is of CPMV-**5**; other conjugates show chromatograms that are essentially identical, unless indicated otherwise. Note the trace monitored at 496 nm, showing fluorescein covalently bound to CPMV. Substrate loadings were calculated using the 496 nm absorbance values. SDS-PAGE analysis of all conjugates was also performed. Figure 10 shows size-exclusion FPLC traces of CPMV-**5**. Traces were monitored at 3 different wavelengths. Gels essentially identical to that shown in Figure 8 (lane 2) were obtained for all samples, unless indicated otherwise. The EMAN program was used to measure particle diameter (www.software-ncmi.bcm.tmc.edu/ncmi/homes/steve1/EMAN/doc).

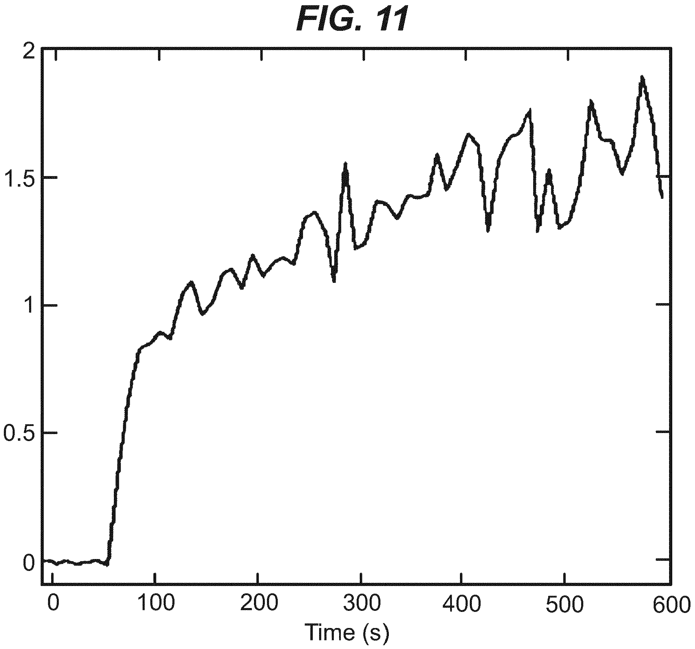

***CPMV-8b.*** The attachment of **8b** to CPMV was further confirmed by monitoring the rate of aggregation of CPMV-**8b** with the dimeric Galectin-4 at 490 nm, where no absorbance of either icosahedral CPMV-**8b** or galectin-4 is observed (Figure 11). Gel formation was rapid at virus concentrations of 1.0 mg/mL. Figure 11 shows a time course of agglutination monitored at 490 nm for a mixture of galectin-4 (300 µg/mL, 50 µL of) and CPMV**-8b** (1.0 mg/mL, 77 µL) in phosphate-buffered saline.

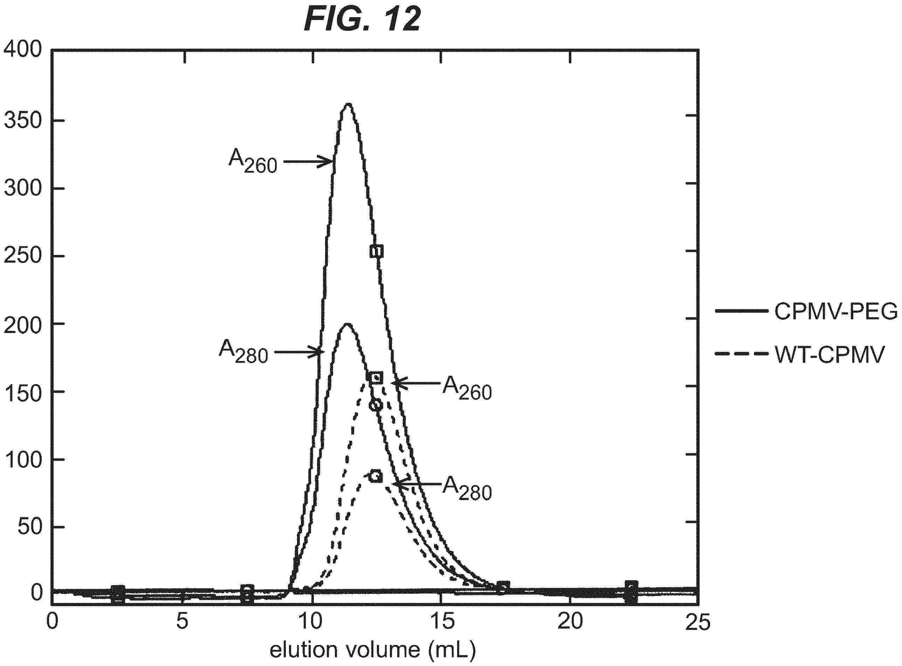

***CPMV-13.*** Analytical size exclusion FPLC of CPMV-**13** is shown in Figure 12. The more rapid elution of CPMV-PEG relative to wild-type CPMV indicates a substantial size increase of the particle. Figure 12 shows size-exclusion FPLC of wild-type CPMV and CPMV-**13.** Protein from disassembled particles would appear at retention times greater than that of the observed peaks. Both samples display A₂₆₀/A₂₈₀ ratios that are characteristic of intact, RNA-containing capsids. The void volume of the column is 10 mL.

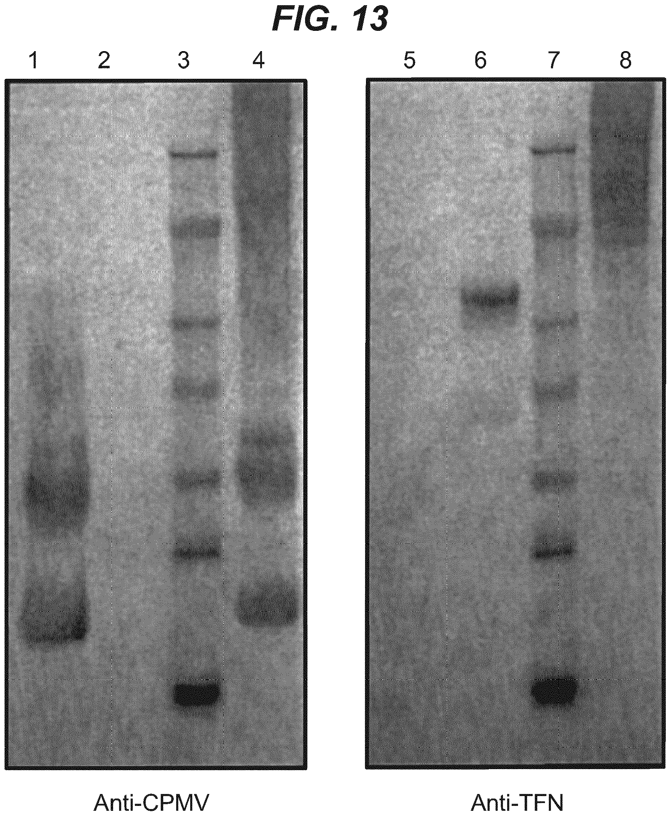

***CPMV-14.*** Western blots of conjugate CPMV-**14** using antibodies against both CPMV and human Tfn show that high molecular weight bands react with both antibodies, indicating modification of CPMV with Tfn (Figure 13). Figure 13 shows Western blots of CPMV-**14** using polyclonal antibodies against CPMV or human Tfn. Proteins denatured on a 4-12% bis-tris gel were transferred to a PVDF membrane and blocked with 5% milk. The membrane was then incubated with antibodies against CPMV (produced by the Manchester laboratory, 1:2000 dilution) or human Tfn (goat, Sigma; 1:2000 dilution). Subsequent incubation of HRP conjugates of goat-anti-rabbit (for anti-CPMV) or rabbit-anti-goat (for anti-Tfn), used at the manufacturer's recommended dilution followed by TMB membrane peroxidase substrate permitted visualization of the protein bands. Samples are as follows: **4** (lanes 1, 5); **14** (lanes 2, 6); molecular weight marker (lanes 3, 7); CPMV-(**14**)ₙ (lanes 4, 8).

### EXAMPLE 14

### Method for Alkyne-Azide Coupling in the Presence of Ruthenium Catalyst

The fundamental process for ruthenium catalyzed alkyne-azide coupling is as shown below. See, for example, J. Am. Chem. Soc., 127: 15998-15999, 2005.

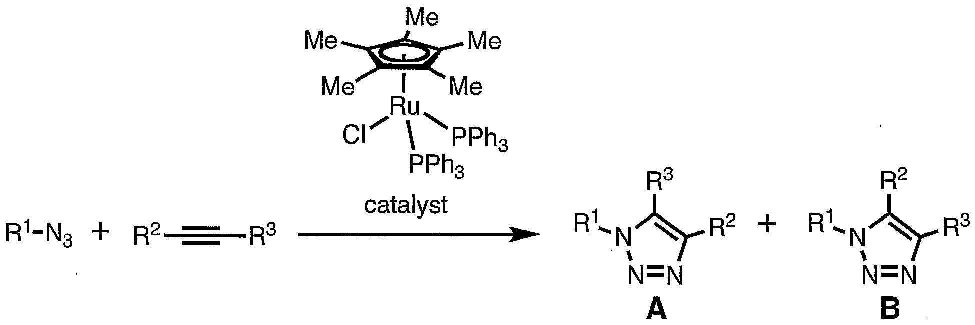

Notable features of the alkyne azide catalysis are that the ruthenium-catalyzed reaction tolerates internal alkynes (R² and R³ both not H), whereas the copper-catalyzed reaction requires R² or R³ to be H. The most active ruthenium catalysts give the opposite regiochemistry to the copper reaction: when R² = H, copper would make product B but Ru makes product A.

In the current realization of the technology, propargylic substrates such as 1 are favorable, reacting faster than many other kinds of alkynes.

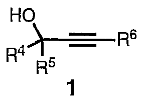

The structure of the ruthenium catalyst above has been shown to have activity in the alkyne azide cycloaddition reaction. Variations on the ruthenium catalyst and other ruthenium containing structures are likely to work as catalysts in alkyne azide cycloaddition reactions for methods of coupling a compound to a scaffold.

All publications and patent applications cited in this specification are herein incorporated by reference in their entirety for all purposes as if each individual publication or patent application were specifically and individually indicated to be incorporated by reference for all purposes.

Although the foregoing invention has been described in some detail by way of illustration and example for purposes of clarity of understanding, it will be readily apparent to one of ordinary skill in the art in light of the teachings of this invention that certain changes and modifications may be made thereto without departing from the spirit or scope of the appended claims.

### <embodiment>

Further numbered embodiments :
1. A method for coupling a compound to a scaffold comprising:
   catalyzing a reaction between at least one terminal alkyne moiety on the compound, and at least one azide moiety on the scaffold forming at least one triazole thereby,
   the catalysis being effected by addition of a metal ion in the presence of a ligand for the metal ion, and
   the scaffold having a plurality of such azide moieties, such that a plurality of compound molecules can be coupled with the scaffold.
2. The method of embodiment 1 wherein the ligand is monodentate, bidentate, or multidentate.
3. The method of embodiment 1, wherein the metal is Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg.
4. The method of embodiment 3, wherein the metal is Mn, Fe, Co, Mo, Tc, Ru, Rh, Pd, W, Re, Os, Ir, Pt, or Au.
5. The method of embodiment 3, wherein the metal is heterogeneous copper, metallic copper, copper oxide, or copper salts.
6. The method of embodiment 1, further comprising catalyzing the reaction by addition of Cu(I).
7. The method of embodiment 1, further comprising catalyzing the reaction by addition of Cu(II) in the presence of a reducing agent for reducing the Cu(II) to Cu(I), *in situ.*
8. The method of embodiment 1, further comprising catalyzing the reaction by addition of Cu(0) in the presence of an oxidizing agent for oxidizing the Cu(0) to Cu(I), *in situ.*
9. The method of embodiment 1, further comprising catalyzing the reaction by addition of ruthenium.
10. The method of embodiment 1, wherein the scaffold is a solid surface, a protein, a nucleoprotein, a protein aggregate, a protein nanoparticle, a nucleoprotein nanoparticle, vault protein or dendrimer.
11. The method of embodiment 10 wherein the protein nanoparticle or nucleoprotein nanoparticle is a virus or viral nanoparticle.
12. The method of embodiment 1, wherein the scaffold is a paramagnetic particle, semiconductor nanoparticle, quantum dot, metal nanoparticle, glass bead, polymer bead, a porous surface, membrane, electrode, porous material, porous fiber-based materials, zeolites, clays, or controlled-pore glass.
13. The method of embodiment 1 further comprising coupling a multiplicity of compound molecules per scaffold.
14. The method of embodiment 11 further comprising coupling a multiplicity of compound molecules per viral nanoparticle.
15. The method of embodiment 14, further comprising coupling 100 or more compound molecules per viral nanoparticle.
16. The method of embodiment 14, further comprising coupling 150 or more compound molecules per viral nanoparticle.
17. The method of embodiment 14, further comprising coupling 200 or more compound molecules per viral nanoparticle.
18. The method of embodiment 11, wherein the viral nanoparticle is a cowpea mosaic virus nanoparticle.
19. The method of embodiment 1, wherein the compound is a small molecule, a metal complex, a polymer, a carbohydrate, a protein, or a polynucleotide.
20. The method of embodiment 19, wherein the compound is transferrin, an RGD-containing polypeptide, a protective antigen of anthrax toxin, polyethylene glycol, or folic acid.
21. A method for coupling a compound to a scaffold comprising:
   catalyzing a reaction between at least one azide moiety on the compound, and at least one terminal alkyne moiety on the scaffold forming at least one triazole thereby,
   the catalysis being effected by addition of a metal ion in the presence of a ligand for the metal ion, and
   the scaffold having a plurality of such terminal alkyne moieties, such that a plurality of compound molecules can be coupled with the scaffold.
22. The method of embodiment 21 wherein the ligand is monodentate, bidentate, or multidentate.
23. The method of embodiment 21, wherein the metal is Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg.
24. The method of embodiment 23, wherein the metal is Mn, Fe, Co, Mo, Tc, Ru, Rh, Pd, W, Re, Os, Ir, Pt, or Au.
25. The method of embodiment 23, wherein the metal is heterogeneous copper, metallic copper, copper oxide, or copper salts.
26. The method of embodiment 21, further comprising catalyzing the reaction by addition of Cu(I).
27. The method of embodiment 21, further comprising catalyzing the reaction by addition of Cu(II) in the presence of a reducing agent for reducing the Cu(II) to Cu(I), *in situ.*
28. The method of embodiment 21, further comprising catalyzing the reaction by addition of Cu(0) in the presence of an oxidizing agent for oxidizing the Cu(0) to Cu(I), *in situ.*
29. The method of embodiment 21, further comprising catalyzing the reaction by addition of ruthenium.
30. The method of embodiment 21, wherein the scaffold is a solid surface, a protein, a nucleoprotein, a protein aggregate, a protein nanoparticle, a nucleoprotein nanoparticle, vault protein or dendrimer.
31. The method of embodiment 30 wherein the protein nanoparticle or nucleoprotein nanoparticle is a virus or viral nanoparticle.
32. The method of embodiment 21, wherein the scaffold is a paramagnetic particle, semiconductor nanoparticle, quantum dot, metal nanoparticle, glass bead, polymer bead, a porous surface, membrane, electrode, porous material, porous fiber-based materials, zeolites, clays, or controlled-pore glass.
33. The method of embodiment 21 further comprising coupling a multiplicity of compound molecules per scaffold.
34. The method of embodiment 31 further comprising coupling a multiplicity of compound molecules per viral nanoparticle.
35. The method of embodiment 34, further comprising coupling 100 or more compound molecules per viral nanoparticle.
36. The method of embodiment 34, further comprising coupling 150 or more compound molecules per viral nanoparticle.
37. The method of embodiment 34, further comprising coupling 200 or more compound molecules per viral nanoparticle.
38. The method of embodiment 31, wherein the viral nanoparticle is a cowpea mosaic virus nanoparticle.
39. The method of embodiment 21, wherein the compound is a small molecule, a metal complex, a polymer, a carbohydrate, a protein, or a polynucleotide.
40. The method of embodiment 39, wherein the compound is transferrin, an RGD-containing polypeptide, a protective antigen of anthrax toxin, polyethylene glycol, or folic acid.
41. A method comprising:
   catalyzing a reaction between at least one terminal alkyne moiety on a first reactant and at least one azide moiety on a second reactant forming at least one triazole thereby,
   the catalysis being effected by addition of a metal in the presence of a ligand for the metal ion, and
   the first reactant having a plurality of terminal alkyne moieties such that a plurality of second reactants can be coupled to the first reactant, or the second reactant having a plurality of azide moieties such that a plurality of first reactants can be coupled to the second reactant.
42. The method of embodiment 41 wherein the ligand is monodentate, bidentate, or multidentate.
43. The method of embodiment 41, wherein the metal is Ti, V, Cr, Mn, Fe, Co, Ni, Cu, Zn, Zr, Nb, Mo, Tc, Ru, Rh, Pd, Ag, Cd, Hf, Ta, W, Re, Os, Ir, Pt, Au, or Hg.
44. The method of embodiment 43, wherein the metal is Mn, Fe, Co, Mo, Tc, Ru, Rh, Pd, W, Re, Os, Ir, Pt, or Au.
45. The method of embodiment 43, wherein the metal is heterogeneous copper, metallic copper, copper oxide, or copper salts.
46. The method of embodiment 43, further comprising catalyzing the reaction by addition of Cu(I).
47. The method of embodiment 43, further comprising catalyzing the reaction by addition of Cu(II) in the presence of a reducing agent for reducing the Cu(II) to Cu(I), *in situ.*
48. The method of embodiment 43, further comprising catalyzing the reaction by addition of Cu(0) in the presence of an oxidizing agent for oxidizing the Cu(0) to Cu(I), *in situ.*
49. The method of embodiment 43, further comprising catalyzing the reaction by addition of ruthenium.
50. The method of embodiment 41 wherein the first reactant is a scaffold having a plurality of terminal alkyne moieties for coupling to the second reactant.
51. The method of embodiment 50 wherein the second reactant is a compound with one or more azide moieties.
52. The method of embodiment 41 wherein the second reactant is a scaffold having a plurality of azide moieties for coupling to the first reactant.
53. The method of embodiment 52 wherein the first reactant is a compound with one or more terminal alkyne moieties.

## Claims

1. A method for coupling a compound to a scaffold comprising:
catalyzing a reaction between at least one terminal alkyne moiety on the compound, and at least one azide moiety on the scaffold forming at least one triazole thereby,
the catalysis being effected by addition of a Cu(I) ion in the presence of a ligand for the Cu(I) ion, and
the scaffold having a plurality of such azide moieties, such that a plurality of compound molecules can be coupled to the scaffold, or alternatively comprising:
catalyzing a reaction between at least one azide moiety on the compound, and at least one terminal alkyne moiety on the scaffold forming at least one triazole thereby,
the catalysis being effected by addition of a Cu(I) ion in the presence of a ligand for the Cu(I) ion, and
the scaffold having a plurality of such terminal alkyne moieties, such that a plurality of compound molecules can be coupled to the scaffold.

2. The method of claim 1 wherein the ligand is monodentate, bidentate, or multidentate.

3. The method of claim 1 wherein the Cu(I) ion is formed by the reaction of Cu(II) in the presence of a reducing agent for reducing the Cu(II) to Cu(I), *in situ.*

4. The method of claim 1 wherein the Cu(I) ion is from a Cu(I) salt.

5. The method of claim 1 wherein the Cu(I) ion is formed by the reaction of Cu(0) in the presence of an oxidizing agent for oxidizing the Cu(0) to Cu(I), *in situ.*

6. The method of claim 1 wherein the scaffold is a nucleoprotein nanoparticle, a solid surface, a protein, a nucleoprotein, a protein aggregate, a protein nanoparticle, vault protein or dendrimer.

7. The method of claim 6 wherein the protein nanoparticle or nucleoprotein nanoparticle is a virus or viral nanoparticle.

8. The method of claim 1 wherein the scaffold is a paramagnetic particle, semiconductor nanoparticle, quantum dot, metal nanoparticle, glass bead, polymer bead, a porous surface, membrane, electrode, porous material, porous fiber-based materials, zeolites, clays, or controlled-pore glass.

9. The method of claim 1 wherein a multiplicity of compound molecules is coupled to the scaffold.

10. The method of claim 9 wherein the scaffold is a viral nanoparticle.

11. The method of claim 10 wherein (i) 100 or more compound molecules are coupled to the viral nanoparticles, or (ii) 150 or more compound molecules are coupled to the viral nanoparticles, or (iii) 200 or more compound molecules are coupled to the viral nanoparticles.

12. The method of claim 10 wherein the viral nanoparticle is a cowpea mosaic virus nanoparticle.

13. The method of claim 1 wherein the compound is a small molecule, a metal complex, a polymer, a carbohydrate, a protein, or a polynucleotide.

14. The method of claim 13 wherein the compound is transferrin, an RGD-containing polypeptide, a protective antigen of anthrax toxin, polyethylene glycol, or folic acid.
